# EUROPEAN PATENT SPECIFICATION

(11) **EP 3 069 143 B1**
(45) Date of publication and mention of the grant of the patent: **10.10.2018**
(21) Application number: 14815406.5
(22) Date of filing: 11.11.2014
(51) Int. Cl.: G01N 33/574, C12Q 1/68

(54) **METHOD, ARRAY AND USE THEREOF FOR DETERMINING PANCREATIC CANCER**
VERFAHREN, ANORDNUNG UND VERWENDUNG DAVON FÜR DEN NACHWEIS VON BAUCHSPEICHELDRÜSENKREBS
PROCÉDÉ, RÉSEAU ET LEUR UTILISATION POUR LA DÉTECTION DU CANCER PANCRÉATIQUE

(30) Priority: 11.11.2013 GB 201319878
(43) Date of publication of application: 21.09.2016
(73) Proprietor: Immunovia AB, 223 81 Lund (SE)
(72) Inventor: BORREBAECK, Carl Arne Krister, S-223 63 Lund (SE); WINGREN, Christer Lars Bertil, S-247 32 Södra Sandby (SE); GERDTSSON, Sandström Anna, 374 40 Karlshamn (SE)
(74) Representative: Potter Clarkson LLP
(86) International application number: PCT/GB2014/053340
(87) International publication number: WO 2015/067969

(56) References cited:
- WO-A2-2006/110581
- WO-A2-2012/120288
- US-A1- 2007 212 738
- LI H ET AL: "Expressions of Syk and VEGF-D protein in pancreatic cancer and their clinical significance", CHINESE JOURNAL OF CANCER PREVENTION AND TREATMENT 200808 CN, vol. 15, no. 15, August 2008 (2008-08), pages 1166-1168, XP008175337, ISSN: 1673-5269
- FUJIWARA S I ET AL: "Transforming activity of the lymphotoxin-beta receptor revealed by expression screening", BIOCHEMICAL AND BIOPHYSICAL RESEARCH COMMUNICATIONS, ACADEMIC PRESS INC. ORLANDO, FL, US, vol. 338, no. 2, 16 December 2005 (2005-12-16), pages 1256-1262, XP027218471, ISSN: 0006-291X, DOI: 10.1016/J.BBRC.2005.10.080 [retrieved on 2005-11-09]
- C. WINGREN ET AL: "Identification of Serum Biomarker Signatures Associated with Pancreatic Cancer", CANCER RESEARCH, vol. 72, no. 10, 15 May 2012 (2012-05-15), pages 2481-2490, XP055172675, ISSN: 0008-5472, DOI: 10.1158/0008-5472.CAN-11-2883
- JOHAN INGVARSSON ET AL: "Detection of pancreatic cancer using antibody microarray-based serum protein profiling", PROTEOMICS, vol. 8, no. 11, 1 June 2008 (2008-06-01), pages 2211-2219, XP055172677, ISSN: 1615-9853, DOI: 10.1002/pmic.200701167
- FRIDA PAULY ET AL: "Protein Expression Profiling of Formalin-Fixed Paraffin-Embedded Tissue Using Recombinant Antibody Microarrays", JOURNAL OF PROTEOME RESEARCH, vol. 12, no. 12, 24 September 2013 (2013-09-24), pages 5943-5953, XP055194718, ISSN: 1535-3893, DOI: 10.1021/pr4003245
- CHO J H ET AL: "W1412 Expression of Sox-11 and Sox-4 Is Involved in Pathogenesis of Solid Pseudopapillary Tumor in Pancreas", GASTROENTEROLOGY, ELSEVIER, PHILADELPHIA, PA, vol. 134, no. 4, 1 April 2008 (2008-04-01) , pages A-699, XP023435059, ISSN: 0016-5085, DOI: 10.1016/S0016-5085(08)63262-X [retrieved on 2008-04-01]

## Description

### Field of Invention

The present invention relates to methods for detecting pancreatic cancer, and biomarkers and arrays for use in the same.

### Background

Pancreatic ductal adenocarcinoma, or pancreatic cancer (PC) is the 4th most common cancer-related cause of death, resulting in almost as many deaths as in breast cancer in the United States, despite a 10 times lower incidence [1]. The poor prognosis is mostly due to the inability to detect PC at an early stage, even though data support that it takes more than five years from tumor initiation until the acquisition of metastatic ability [2], clearly demonstrating a window of opportunity for early detection if markers were available. Today, the cancer is often detected at advanced disease progression, with tumors that are inoperable and already have metastasized [1, 3]. However, even if the five-year survival of large resected tumors is only 10-20% [4, 5], it increases to 30-60% if tumors <20 mm can be resected [6, 7], and to >75% if the size at resection is <10 mm [8]. The late detection is due to unspecific clinical symptoms as well as lack of sensitive technologies and markers for early diagnosis. Interestingly, studies suggest that pancreatic tumors could be resectable as early as six months prior to clinical diagnosis at an asymptomatic stage [9, 10].

The so far most evaluated marker for PC, CA19-9, suffers from poor specificity, with elevated levels also in pancreatitis and other cancers, and a complete absence in Lewis a and b negative tumors. Consequently, the use of CA19-9 for pancreatic cancer screening has been discouraged [11]. Despite many efforts, no other single biomarkers have been shown to outperform CA19-9, and in recent years the field has been moving towards multiplexed marker panels for increased sensitivity and specificity [12]. Panels have primarily consisted of high abundant blood proteins, frequently acute-phase reactants (e.g. CRP and SAA), known tumor markers (e.g. CA242, CA125 and CEA), adhesion molecules (e.g. ICAM-1 and ADCAM), proteins involved in extracellular matrix degradation (e.g. MMPs and TIMP1), lipoproteins (e.g. Apo-C1, Apo-A2), and several others, most often in combination with CA19-9 [15-20]. Despite reports of high sensitivity for PC versus healthy controls or benign pancreatic conditions, none of these panels have yet been validated for clinical use.

Besides the markers already mentioned, several immunoregulatory proteins may be of interest as tumor biomarkers, as the association between cancer and inflammation keeps unraveling [21]. Considering the systemic effect as well as the multitude in functions of many of the immunoregulatory proteins, it is generally considered that small panels of 2 to 5 markers will not be sufficiently specific for pancreatic cancer, particularly when trying to discriminate pancreatic cancer from pancreatitis and other conditions that present with similar symptoms. However, previous studies have shown that an increased number of these analytes (≤25) may yield highly disease specific immunosignatures reflecting the systemic response to disease [22-24].

Li et al., 2008, Chinese J., Cancer Prevention & Treatment, 15(15): 1166-1168 relates to a method for determining the presence of pancreatic cancer in an individual comprising or consisting of the steps of: (a) providing a sample to be tested from the individual, and (b) determining a biomarker signature of the test sample by measuring the expression in the test sample of two or more biomarkers selected from the group defined in Table III, wherein the expression in the test sample of two or more biomarkers selected from the group defined in Table III is indicative of the individual having pancreatic cancer.

US 2007/212738 A1 (Haley et al.,) relates to diagnostic and prognostic methods for predicting the effectiveness of treatment of a cancer patient with an EGFR kinase inhibitor.

WO 2006/110581 A2 (Chiron Corp (US) & Sagres Discovery Inc.) relates to methods for detecting cancer or the likelihood of developing cancer based on the presence or absence of expression of certain genes or proteins encoded by those genes.

Fujiwara et al., 2005, Biochem. & Biophys. Res. Com., 338(2):1256-1262 discloses that the full-length (wild-type) LTBR protein was found to manifest transforming activity.

Wingren et al., 2012, Cancer Res., 72(10):2481-2490 discloses a prevalidated, multiplexed serum biomarker signature for diagnosis of pancreatic cancer that may improve diagnosis and prevention in premalignant diseases and in screening of high-risk individuals.

Ingvarsson et al., 2008, Proteomics, 8(11):2211-2219 relates to the identification of a protein signature based on 19 nonredundant analytes, that discriminates between cancer patients and healthy subjects.

Pauly et al., 2013, Journal of Proteome Research, 12(12): 5943-5953 shows that proteins could be extracted from formalin-fixed, paraffin-embedded (FFPE) tissue material, labeled, and subsequently profiled in a multiplex, sensitive, and reproducible manner using recombinant scFv antibody microarrays.

Cho et al., 2008, Gastroenterology. 134(4): A-699 discloses the expression of SOX11 and SOX4 in solid-pseudopapillary tumour of the pancreas, but not in normal pancreas or pancreatic adenocarcinoma.

WO 2012/120288 (Immunovia AB) relates to a method for determining the presence of pancreatic cancer in an individual comprising or consisting of the steps of: (a) providing a sample to be tested from the individual, and (b) determining a biomarker signature of the test sample by measuring the expression in the test sample of two or more biomarkers selected from the group defined in Table III, wherein the expression in the test sample of two or more biomarkers selected from the group defined in Table III is indicative of the individual having pancreatic cancer.

Nevertheless, analysis of the immunoregulatory proteome is associated with several challenges. First, the serum concentration of the proteins of interest displays a vast dynamic range, from high microgram to low picogram per mL, which complicates their simultaneous detection using conventional proteomic methodologies [25, 26]. Second, there seems to be a consensus in that promising cancer markers are more likely to be found among the most low abundant, often low-molecular weight proteins [27, 28]. Third, the disease-associated changes in serum levels of these low abundant analytes are expected to be small, and thus a significant number of clinical samples will be needed for adequate statistics [29]. For these purposes, we have designed highly multiplexed recombinant antibody microarrays with close to 300 scFv antibodies targeting mainly immunoregulatory proteins [30]. With these arrays, protein expression can be measured in hundreds of samples in a highly reproducible and high-throughput manner.

### Summary of the Invention

The invention is defined by the claims herein.

In this multicenter study, 338 individual serum samples from patients with pancreatic cancer, benign pancreatic disease, as well as normal controls, were analyzed on our in-house designed antibody microarrays. To define the most discriminative marker signatures, we applied an iterative backward elimination procedure, based on support vector machine analysis and designed to predict the optimal combination of antibodies [31]. To this end, 25-plex immunosignatures for discriminating PC from benign and healthy controls were identified in training sets, prevalidated in independent test sets, and compared to signatures derived from differential protein expression analysis. In addition, protein profiling could be applied to stratify serum samples based on the original location of the tumor in the pancreas, which to the best of our knowledge has not previously been done with proteomics. Together, these findings add important information to the proteome puzzle of pancreatic cancer, which may in the end result in multiplexed biomarkers providing benefit for thousands of patients.

Accordingly, a first aspect of the invention provides a method for determining the presence of pancreatic cancer in an individual comprising or consisting of the steps of:
a) providing a sample to be tested obtained from the individual;
b) determining a biomarker signature of the test sample by measuring the expression in the test sample of two or more biomarkers selected from the group defined in Table A;
wherein step (b) comprises measuring the expression in the test sample of HADH2, and
wherein the expression in the test sample of the two or more biomarkers selected from the group defined in Table A is indicative of the presence of pancreatic cancer.

By "sample to be tested", "test sample" or "control sample" we include a tissue or fluid sample which has been previously taken or derived from an individual. Preferably the sample to be tested is from a mammal. The mammal may be any domestic or farm animal. Preferably, the mammal is a rat, mouse, guinea pig, cat, dog, horse or a primate. Most preferably, the mammal is human. Preferably the sample is a cell or
tissue sample (or derivative thereof) comprising or consisting of plasma, plasma cells, serum, tissue cells or equally preferred, protein or nucleic acid derived from a cell or tissue sample. Preferably test and control samples are derived from the same species.

In one embodiment, the tissue sample is pancreatic tissue. In one embodiment, the cell sample is a sample of pancreatic cells.

By "expression" we mean the level or amount of a gene product such as mRNA or protein.

Methods of detecting and/or measuring the concentration of protein and/or nucleic acid are well known to those skilled in the art, see for example Sambrook and Russell, 2001, Cold Spring Harbor Laboratory Press.

By "biomarker" we mean a naturally-occurring biological molecule, or component or fragment thereof, the measurement of which can provide information useful in the prognosis of pancreatic cancer. For example, the biomarker may be a naturally-occurring protein or carbohydrate moiety, or an antigenic component or fragment thereof.

In one embodiment, the method comprises or consists of steps (a) and (b) and the further steps of:
c) providing a control sample obtained from an individual not afflicted with pancreatic cancer;
d) determining a biomarker signature of the control sample by measuring the expression in the control sample of the two or more biomarkers measured in step (b);
wherein the presence of pancreatic cancer is identified in the event that the expression in the test sample of the two or more biomarkers measured in step (b) is different from the expression in the control sample of the two or more biomarkers measured in step (d).

The one or more control sample may be from a healthy individual (i.e., an individual unafflicted by any disease or condition), an individual afflicted with a non-pancreatic disease or condition or an individual afflicted with a benign pancreatic disease or condition (for example, acute or chronic pancreatitis).

In another embodiment, the method comprises or consists of steps (a) and (b) and, optionally, steps (c) and (d) and the additional steps of:
e) providing one or more control sample obtained from an individual afflicted with pancreatic cancer;
f) determining a biomarker signature of the control sample by measuring the expression in the control sample of the two or more biomarkers measured in step (b);
wherein the presence of pancreatic cancer is identified in the event that the expression in the test sample of the two or more biomarkers measured in step (b) corresponds to the expression in the control sample of the two or more biomarkers measured in step (f).

In one embodiment, a standard or reference value is used instead of, or in addition, to the one or more positive or negative control. Hence, the standard or references value(s) may be determined in separate procedures from the test value(s).

In one embodiment, the method is for determining the presence of pancreatic cancer originating from (i) the head of the pancreas or (ii) the body or tail of the pancreas. In this embodiment, step (e) may comprise (i) providing one or more control sample from an individual afflicted with pancreatic cancer originating from the head of the pancreas and/or (ii) providing one or more control sample from an individual afflicted with pancreatic cancer originating from the body or tail of the pancreas.

In one embodiment, the presence of pancreatic cancer originating from head of the pancreas is identified in the event that the expression in the (e)(i) test sample (where present) of the two or more biomarkers measured in step (b) corresponds to the expression in the control sample of the two or more biomarkers measured in step (f) and/or the expression in the (e)(ii) test sample (where present) of the two or more biomarkers measured in step (b) is different from the expression in the control sample of the two or more biomarkers measured in step (f).

In one embodiment, the presence of pancreatic cancer originating from the body or tail of the pancreas is identified in the event that the expression in the (e)(i) test sample (where present) of the two or more biomarkers measured in step (b) is different from the expression in the control sample of the two or more biomarkers measured in step (f) and/or the expression in the (e)(ii) test sample (where present) of the two or more biomarkers measured in step (b) corresponds to the expression in the control sample of the two or more biomarkers measured in step (f).

By "corresponds to the expression in the control sample" we include that the expression of the two or more biomarkers in the sample to be tested is the same as or similar to the expression of the two or more biomarkers of the positive control sample. Preferably the expression of the two or more biomarkers in the sample to be tested is identical to the expression of the two or more biomarkers of the positive control sample.

Differential expression (up-regulation or down regulation) of biomarkers, or lack thereof, can be determined by any suitable means known to a skilled person. Differential expression is determined to a p value of at least less than 0.05 (p = < 0.05), for example, at least <0.04, <0.03, <0.02, <0.01, <0.009, <0.005, <0.001, <0.0001, <0.00001 or at least <0.000001. Preferably, differential expression is determined using a support vector machine (SVM). Preferably, the SVM is an SVM as described below. Most preferably, the SVM is the SVM described in Table B, below.

It will be appreciated by persons skilled in the art that differential expression may relate to a single biomarker or to multiple biomarkers considered in combination (*i.e.* as a biomarker *signature*). Thus, a p value may be associated with a single biomarker or with a group of biomarkers. Indeed, proteins having a differential expression p value of greater than 0.05 when considered individually may nevertheless still be useful as biomarkers in accordance with the methods of the invention when their expression levels are considered in combination with one or more other biomarkers.

As exemplified in the accompanying examples, the expression of certain proteins in a tissue, blood, serum or plasma test sample may be indicative of pancreatic cancer in an individual. For example, the relative expression of certain serum proteins in a single test sample may be indicative of the presence of pancreatic cancer in an individual.

Step (b) may further comprise measuring the expression of 1 or more biomarker from the biomarkers listed in Table A(i). Step (b) may comprise or consist of measuring the expression of each of the biomarkers listed in Table A(i), for example at least 2 of the biomarkers listed in Table A(i). Hence, step (b) may comprise or consist of measuring the expression of all of the biomarkers listed in Table A(i).

Step (b) may further comprise measuring the expression of 1 or more biomarker from the biomarkers listed in Table A(ii), for example at least 2, 3, 4, 5, 6, 7, 8 or 9 of the biomarkers listed in Table A(ii). Hence, step (b) may comprise measuring the expression of all of the biomarkers listed in Table A(ii).

Step (b) may further comprise measuring the expression of 1 or more biomarker from the biomarkers listed in Table A(iii), for example at least 2, 3, 4, 5, 6, 7, 8, 9, 10, 11, 12, 13, 14, 15, 16 or 17 of the biomarkers listed in Table A(iii). Hence, step (b) may comprise measuring the expression of all of the biomarkers listed in Table A(iii).

Step (b) may further comprise measuring the expression of 1 or more biomarker from the biomarkers listed in Table A(iv), for example at least 2, 3, 4, 5, 6, 7, 8, 9, 10, 11, 12, 13, 14, 15, 16, 17, 18, 19, 20, 21 or 22, 23 or 24 of the biomarkers listed in Table A(iv). Hence, step (b) may comprise measuring the expression of all of the biomarkers listed in Table A(iv).

Step (b) may further comprise measuring the expression of 1 or more biomarker from the biomarkers listed in Table A(v), for example at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55 or 56 of the biomarkers listed in Table A(v). Hence, step (b) may comprise measuring the expression of all of the biomarkers listed in Table A(v).

Step (b) may further comprise measuring the expression of 1 or more biomarker from the biomarkers listed in Table A(vi). Hence, step (b) may comprise measuring the expression of all of the biomarkers listed in Table A(vi).

Step (b) may further comprise measuring the expression of 1 or more biomarker from the biomarkers listed in Table A, for example at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109 or 110 of the biomarkers listed in Table A. Hence, step (b) may comprise measuring the expression of all of the biomarkers listed in Table A.

Where the method is for determining the presence of pancreatic adenocarcinoma step (b) preferably comprises measuring the expression of HADH2 and:
1 or more biomarkers from the biomarkers listed in Table A(i), for example at least 2 of the biomarkers listed in Table A(i);
1 or more biomarker from the biomarkers listed in Table A(ii), for example at least 2, 3, 4, 5, 6, 7, 8 or 9 of the biomarkers listed in Table A(ii);
1 or more biomarker from the biomarkers listed in Table A(iii), for example at least 2, 3, 4, 5, 6, 7, 8, 9, 10, 11, 12, 13, 14, 15, 16 or 17 of the biomarkers listed in Table A(iii);
1 or more biomarker from the biomarkers listed in Table A(iv), for example at least 2, 3, 4, 5, 6, 7, 8, 9, 10, 11, 12, 13, 14, 15, 16, 17, 18, 19, 20, 21, 22, 23 or 24 of the biomarkers listed in Table A(iv); and/or
1 or more biomarker from the biomarkers listed in Table A(v), for example at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55 or 56 of the biomarkers listed in Table A(v).
1 or more biomarker from the biomarkers listed in Table A(vi). Hence, step (b) may comprise or consist of measuring the expression of all of the biomarkers listed in Table A(vi).

Where the method is for determining the presence of pancreatic adenocarcinoma originating from (i) the head of the pancreas or (ii) the body or tail of the pancreas step (b) preferably comprises measuring the expression of HADH2 and:
1 or more biomarker from the biomarkers listed in Table A(ii), for example at least 2, 3, 4, 5, 6, 7, 8 or 9 of the biomarkers listed in Table A(ii);
1 or more biomarker from the biomarkers listed in Table A(iv), for example at least 2, 3, 4, 5, 6, 7, 8, 9, 10, 11, 12, 13, 14, 15, 16, 17, 18, 19, 20, 21, 22, 13 or 24 of the biomarkers listed in Table A(iv); and/or
1 or more biomarker listed in Table A(vi).

When referring to a "normal" disease state we include individuals not afflicted with chronic pancreatitis (ChP) or acute inflammatory pancreatitis (AIP). Preferably the individuals are not afflicted with any pancreatic disease or disorder. Most preferably, the individuals are healthy individuals, i.e., they are not afflicted with any disease or disorder.

In the methods of the invention, step (b) comprises measuring the expression in the test sample of HADH2 and at least one additional biomarker selected from the group defined in Table A (i), (ii) or (iii)

Thus, in one embodiment, step (b) comprises measuring the expression of Transcription factor SOX-11. In an additional embodiment, step (b) comprises measuring the expression of Integrin alpha-10. In an additional embodiment, step (b) comprises measuring the expression of EDFR. In an additional embodiment, step (b) comprises measuring the expression of EPFR. In an additional embodiment, step (b) comprises measuring the expression of LSADHR. In an additional embodiment, step (b) comprises measuring the expression of SEAHLR. In an additional embodiment, step (b) comprises measuring the expression of AQQHQWDGLLSYQDSLS. In an additional embodiment, step (b) comprises measuring the expression of WTRNSNMNYWLIIRL. In an additional embodiment, step (b) comprises measuring the expression of WDSR. In an additional embodiment, step (b) comprises measuring the expression of DFAEDK. In an additional embodiment, step (b) comprises measuring the expression of FASN protein. In an additional embodiment, step (b) comprises measuring the expression of GAK protein. In an additional embodiment, step (b) comprises measuring the expression of LNVWGK. In an additional embodiment, step (b) comprises measuring the expression of LTEFAK. In an additional embodiment, step (b) comprises measuring the expression of LYEIAR. In an additional embodiment, step (b) comprises measuring the expression of Megakaryocyte-associated tyrosine-protein kinase. In an additional embodiment, step (b) comprises measuring the expression of Oxysterol-binding protein-related protein 3. In an additional embodiment, step (b) comprises measuring the expression of QEASFK. In an additional embodiment, step (b) comprises measuring the expression of SSAYSR. In an additional embodiment, step (b) comprises measuring the expression of QEASFK. In an additional embodiment, step (b) comprises measuring the expression of TEEQLK. In an additional embodiment, step (b) comprises measuring the expression of TLYVGK. In an additional embodiment, step (b) comprises measuring the expression of FLLMQYGGMDEHAR. In an additional embodiment, step (b) comprises measuring the expression of GIVKYLYEDEG. In an additional embodiment, step (b) comprises measuring the expression of GIVKYLYEDEG. In an additional embodiment, step (b) comprises measuring the expression of Tumor necrosis factor receptor superfamily member 3. In an additional embodiment, step (b) comprises measuring the expression of Tyrosine-protein kinase SYK. In an additional embodiment, step (b) comprises measuring the expression of Angiomotin. In an additional embodiment, step (b) comprises measuring the expression of C-C motif chemokine 2. In an additional embodiment, step (b) comprises measuring the expression of C-C motif chemokine 5. In an additional embodiment, step (b) comprises measuring the expression of CD40 ligand. In an additional embodiment, step (b) comprises measuring the expression of Glucagon-like peptide-1. In an additional embodiment, step (b) comprises measuring the expression of Immunoglobilin M. In an additional embodiment, step (b) comprises measuring the expression of Interleukin-1 alpha. In an additional embodiment, step (b) comprises measuring the expression of Interleukin-1 receptor antagonist protein. In an additional embodiment, step (b) comprises measuring the expression of Interleukin-11. In an additional embodiment, step (b) comprises measuring the expression of Interleukin-12. In an additional embodiment, step (b) comprises measuring the expression of Interleukin-16. In an additional embodiment, step (b) comprises measuring the expression of Interleukin-18. In an additional embodiment, step (b) comprises measuring the expression of Interleukin-2. In an additional embodiment, step (b) comprises measuring the expression of Interleukin-3. In an additional embodiment, step (b) comprises measuring the expression of Interleukin-4. In an additional embodiment, step (b) comprises measuring the expression of Interleukin-6. In an additional embodiment, step (b) comprises measuring the expression of Interleukin-7. In an additional embodiment, step (b) comprises measuring the expression of Interleukin-9. In an additional embodiment, step (b) comprises measuring the expression of Lewis x. In an additional embodiment, step (b) comprises measuring the expression of Lymphotoxin-alpha. In an additional embodiment, step (b) comprises measuring the expression of Transforming growth factor beta-1. In an additional embodiment, step (b) comprises measuring the expression of Vascular endothelial growth factor. In an additional embodiment, step (b) comprises measuring the expression of Visual system homeobox 2. In an additional embodiment, step (b) comprises measuring the expression of HLA-DR/DP. In an additional embodiment, step (b) comprises measuring the expression of Apolipoprotein A1. In an additional embodiment, step (b) comprises measuring the expression of Apolipoprotein A4. In an additional embodiment, step (b) comprises measuring the expression of Apolipoprotein B-100. In an additional embodiment, step (b) comprises measuring the expression of ATP synthase subunit beta, mitochondrial. In an additional embodiment, step (b) comprises measuring the expression of Beta-galactosidase. In an additional embodiment, step (b) comprises measuring the expression of Cathepsin W. In an additional embodiment, step (b) comprises measuring the expression of C-C motif chemokine 13. In an additional embodiment, step (b) comprises measuring the expression of C-C motif chemokine 7. In an additional embodiment, step (b) comprises measuring the expression of CD40 protein. In an additional embodiment, step (b) comprises measuring the expression of Complement C1q. In an additional embodiment, step (b) comprises measuring the expression of Complement C1s. In an additional embodiment, step (b) comprises measuring the expression of Complement C3. In an additional embodiment, step (b) comprises measuring the expression of Complement C4. In an additional embodiment, step (b) comprises measuring the expression of Complement C5. In an additional embodiment, step (b) comprises measuring the expression of Complement factor B. In an additional embodiment, step (b) comprises measuring the expression of Cyclin-dependent kinase 2. In an additional embodiment, step (b) comprises measuring the expression of Cystatin-C. In an additional embodiment, step (b) comprises measuring the expression of Eotaxin. In an additional embodiment, step (b) comprises measuring the expression of Epidermal growth factor receptor. In an additional embodiment, step (b) comprises measuring the expression of Glucagon-like peptide 1 receptor. In an additional embodiment, step (b) comprises measuring the expression of Granulocyte-macrophage colony-stimulating factor. In an additional embodiment, step (b) comprises measuring the expression of Integrin alpha-11. In an additional embodiment, step (b) comprises measuring the expression of Intercellular adhesion molecule 1. In an additional embodiment, step (b) comprises measuring the expression of Interferon gamma. In an additional embodiment, step (b) comprises measuring the expression of Interleukin-1 beta. In an additional embodiment, step (b) comprises measuring the expression of Interleukin-10. In an additional embodiment, step (b) comprises measuring the expression of Interleukin-13. In an additional embodiment, step (b) comprises measuring the expression of Interleukin-5. In an additional embodiment, step (b) comprises measuring the expression of Interleukin-8. In an additional embodiment, step (b) comprises measuring the expression of Keratin, type I cytoskeletal 19. In an additional embodiment, step (b) comprises measuring the expression of Leptin. In an additional embodiment, step (b) comprises measuring the expression of Lumican. In an additional embodiment, step (b) comprises measuring the expression of Mitogen-activated protein kinase 1. In an additional embodiment, step (b) comprises measuring the expression of Mitogen-activated protein kinase 8. In an additional embodiment, step (b) comprises measuring the expression of Mucin-1. In an additional embodiment, step (b) comprises measuring the expression of Myomesin-2. In an additional embodiment, step (b) comprises measuring the expression of Osteopontin. In an additional embodiment, step (b) comprises measuring the expression of Phosphatidylinositol 3-kinase regulatory subunit alpha. In an additional embodiment, step (b) comprises measuring the expression of Plasma protease C1 inhibitor. In an additional embodiment, step (b) comprises measuring the expression of Properdin. In an additional embodiment, step (b) comprises measuring the expression of Prostate-specific antigen. In an additional embodiment, step (b) comprises measuring the expression of Receptor tyrosine-protein kinase erbB-2. In an additional embodiment, step (b) comprises measuring the expression of Regulator of nonsense transcripts 3B. In an additional embodiment, step (b) comprises measuring the expression of Ribosomal protein S6 kinase alpha-2. In an additional embodiment, step (b) comprises measuring the expression of Sialyl Lewis x. In an additional embodiment, step (b) comprises measuring the expression of Signal-transducing adaptor protein 2. In an additional embodiment, step (b) comprises measuring the expression of SUMO-conjugating enzyme UBC9. In an additional embodiment, step (b) comprises measuring the expression of TBC1 domain family member 9. In an additional embodiment, step (b) comprises measuring the expression of Transmembrane peptide. In an additional embodiment, step (b) comprises measuring the expression of Tumor necrosis factor alpha. In an additional embodiment, step (b) comprises measuring the expression of Tumor necrosis factor receptor superfamily member 14. In an additional embodiment, step (b) comprises measuring the expression of Tyrosine-protein kinase BTK. In an additional embodiment, step (b) comprises measuring the expression of Tyrosine-protein kinase JAK3. In an additional embodiment, step (b) comprises measuring the expression of Tyrosine-protein phosphatase non-receptor type 1. In an additional embodiment, step (b) comprises measuring the expression of Ubiquitin carboxyl-terminal hydrolase isozyme L5. In an additional embodiment, step (b) comprises measuring the expression of Ubiquitin-conjugating enzyme E2 C. In an additional embodiment, step (b) comprises measuring the expression of FIQTDK.

By "transmembrane peptide" or "TM peptide" we mean a peptide derived from a 10TM protein, to which the scFv antibody construct of SEQ ID NO: 1 below has specificity (wherein the CDR sequences are indicated by bold, italicised text): 
MAEVQLLESGGGLVQPGGSLRLSCAASGFTFSSYGFHWVRQAPGKGLEWVSLISWDG
GSTYYADSVKGRFTISRDNSKNTLYLQMNSLRAEDTAVYYCARGTWFDPWGQGTLVT
VSSGGGGSGGGGSGGGGSQSVLTQPPSASGTPGQRVTISCSGSSSNIGNNAVNWYQ
 
QLPGTAPKLLIYRNNQRPSGVPDRFSGSKSGTSASLAISGLRSEDEADYYCAAWDDSL
SWVFGGGTKLTVLG
 **[SEQ ID NO: 1]**

Hence, this scFv may be used or any antibody, or antigen binding fragment thereof, that competes with this scFv for binding to the 10TM protein. For example, the antibody, or antigen binding fragment thereof, may comprise the same CDRs as present in SEQ ID NO:1.

It will be appreciated by persons skilled in the art that such an antibody may be produced with an affinity tag (*e.g.* at the C-terminus) for purification purposes. For example, an affinity tag of SEQ ID NO: 2 below may be utilised:
DYKDHDGDYKDHDIDYKDDDDKAAAHHHHHH
**[SEQ ID NO: 2]**

In an additional embodiment, the two or more biomarkers measured in step (b) comprise HADH2 and one or more biomarker selected from the group consisting of:
Angiomotin, Apo-A1, Apo-A4, ATP-5B, BTK, C1 inh, C1q, C3, C5, CD40, CD40L, Cystatin C, Eotaxin, Factor B, GAK, GM-CSF, IL-11, IL-13, IL-3, IL-4, IL-6, IL-8, IL-9, KSYK-1, LDL, MAPK1, MCP-1, PTP-1B, Sialyl Lewis x, Sox11A, TGF-beta1, TNF-alpha, TNFRSF3, UCHL5 and UPF3B.

In this embodiment, the method may be for discriminating between pancreatic cancer (PC), and non-pancreatic cancer (NC) and/or benign pancreatic conditions (BC).

In an additional embodiment the two or more biomarkers measured in step (b) comprise HADH2 and one or more biomarker selected from the group consisting of:
Angiomotin, ATP-5B, C1 inh, C1q, C3, C5, CD40, Cystatin C, Eotaxin, Factor B, GAK, IL-11, IL-13, IL-6, IL-8, LDL and TNF-alpha.

In this embodiment, the method may be for discriminating between pancreatic cancer (PC), and non-pancreatic cancer (NC).

In an additional embodiment, the two or more biomarkers measured in step (b) comprise HADH2 and one or more biomarker selected from the group consisting of:
Apo-A1, Apo-A4, BTK, C1 inh, C5, CD40L, CIMS, Factor B, GM-CSF, HADH2, IL-3, IL-4, IL-4, IL-9, KSYK-1, MAPK1, MCP-1, PTP-1B, Sialyl Lewis x, Sox11A, TGF-beta1, TNFRSF3, UCHL5 and UPF3B.

In this embodiment, the method may be for discriminating between pancreatic cancer (PC), and benign pancreatic conditions (BC).

In an additional embodiment, the method comprises steps (a) and (b), optionally, steps (c) and (d), optionally, steps (e) and (f), and the additional step of:
g) determining the presence of pancreatic cancer based on the expression of the two or more biomarkers measured in step (b).

In an additional embodiment, the method is based on the trends (up- or down-regulation) identified in Table 2.

In an additional embodiment step (b) and, where present, steps (d) and (f) are performed by contacting the sample to be tested with binding moiety for the two or more biomarkers measured in step (b), for example, as first binding moiety as defined below.

In an additional embodiment, the method comprises or consists of the *in vitro* use of biomarkers listed in Table 3.

Generally, diagnosis is made with an ROC AUC of at least 0.55, for example with an ROC AUC of at least 0.60, 0.65, 0.70, 0.75, 0.80, 0.85, 0.90, 0.95, 0.96, 0.97, 0.98, 0.99 or with an ROC AUC of 1.00. Preferably, diagnosis is made with an ROC AUC of at least 0.85, and most preferably with an ROC AUC of 1.

Typically, diagnosis is performed using a support vector machine (SVM), such as those available from http://cran.r-project.org/web/packages/e1071/index.html (e.g. e1071 1.5-24). However, any other suitable means may also be used.

Support vector machines (SVMs) are a set of related supervised learning methods used for classification and regression. Given a set of training examples, each marked as belonging to one of two categories, an SVM training algorithm builds a model that predicts whether a new example falls into one category or the other. Intuitively, an SVM model is a representation of the examples as points in space, mapped so that the examples of the separate categories are divided by a clear gap that is as wide as possible. New examples are then mapped into that same space and predicted to belong to a category based on which side of the gap they fall on.

More formally, a support vector machine constructs a hyperplane or set of hyperplanes in a high or infinite dimensional space, which can be used for classification, regression or other tasks. Intuitively, a good separation is achieved by the hyperplane that has the largest distance to the nearest training datapoints of any class (so-called functional margin), since in general the larger the margin the lower the generalization error of the classifier. For more information on SVMs, see for example, Burges, 1998, Data Mining and Knowledge Discovery, 2:121-167.

The SVM may be 'trained' prior to performing the methods of the invention using biomarker profiles from individuals with known disease status (for example, individuals known to have pancreatic cancer, individuals known to have acute inflammatory pancreatitis, individuals known to have chronic pancreatitis or individuals known to be healthy). By running such training samples, the SVM is able to learn what biomarker profiles are associated with pancreatic cancer. Once the training process is complete, the SVM is then able whether or not the biomarker sample tested is from an individual with pancreatic cancer.

However, this training procedure can be by-passed by pre-programming the SVM with the necessary training parameters. For example, diagnoses can be performed according to the known SVM parameters using the SVM algorithm detailed in Table B, based on the measurement of any or all of the biomarkers listed in Table A.

It will be appreciated by skilled persons that suitable SVM parameters can be determined for any combination of the biomarkers listed in Table A by training an SVM machine with the appropriate selection of data (i.e. biomarker measurements from individuals with known pancreatic cancer status). Alternatively, the Table 2 and 3 data may be used to determine a particular pancreatic cancer-associated disease state according to any other suitable statistical method known in the art.

Preferably, the method disclosed has an accuracy of at least 60%, for example 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% accuracy.

Preferably, the method disclosed has a sensitivity of at least 60%, for example 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% sensitivity.

Preferably, the method disclosed has a specificity of at least 60%, for example 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% specificity.

By "accuracy" we mean the proportion of correct outcomes of a method, by "sensitivity" we mean the proportion of all PaC positive sample that are correctly classified as positives, and by "specificity" we mean the proportion of all PaC negative samples that are correctly classified as negatives.

In an additional embodiment, the individual not afflicted with pancreatic cancer is not afflicted with pancreatic cancer (PaC), chronic pancreatitis (ChP) or acute inflammatory pancreatitis (AIP). More preferably, the healthy individual is not afflicted with any pancreatic disease or condition. Even more preferably, the individual not afflicted with pancreatic cancer is not afflicted with any disease or condition. Most preferably, the individual not afflicted with pancreatic cancer is a healthy individual. By a "healthy individual" we include individuals considered by a skilled person to be physically vigorous and free from physical disease.

However, in another embodiment the individual not afflicted with pancreatic cancer is afflicted with chronic pancreatitis. In still another embodiment, the individual not afflicted with pancreatic cancer is afflicted with acute inflammatory pancreatitis.

As previously mentioned the present method is for determining the presence of pancreatic cancer in an individual. In an alternative or additional embodiment the pancreatic cancer is selected from the group consisting of adenocarcinoma, adenosquamous carcinoma, signet ring cell carcinoma, hepatoid carcinoma, colloid carcinoma, undifferentiated carcinoma, and undifferentiated carcinomas with osteoclast-like giant cells. Preferably, the pancreatic cancer is a pancreatic adenocarcinoma. More preferably, the pancreatic cancer is pancreatic ductal adenocarcinoma, also known as exocrine pancreatic cancer.

In a further embodiment, step (b), (d) and/or step (f) is performed using first binding agents capable of binding to the two or more biomarkers (i.e., using two or more first binding agents, wherein each binding agent is capable of specifically binding to one of the two or more biomarkers). It will be appreciated by persons skilled in the art that the first binding agent may comprise or consist of a single species with specificity for one of the protein biomarkers or a plurality of different species, each with specificity for a different protein biomarker.

Suitable binding agents (also referred to as binding molecules) can be selected from a library, based on their ability to bind a given motif, as discussed below.

At least one type of the binding agents, and more typically all of the types, may comprise or consist of an antibody or antigen-binding fragment of the same, or a variant thereof.

Methods for the production and use of antibodies are well known in the art, for example see *Antibodies:* A Laboratory Manual, 1988, Harlow & Lane, Cold Spring Harbor Press, ISBN-13: 978-0879693145, Using Antibodies: A Laboratory Manual, 1998, Harlow & Lane, Cold Spring Harbor Press, ISBN-13: 978-0879695446 and Making and Using Antibodies: A Practical Handbook, 2006, Howard & Kaser, CRC Press, ISBN-13: 978-0849335280 .

Thus, a fragment may contain one or more of the variable heavy (V_{H}) or variable light (V_{L}) domains. For example, the term antibody fragment includes Fab-like molecules (Better et al (1988) Science 240, 1041); Fv molecules (Skerra et al (1988) Science 240, 1038); single-chain Fv (ScFv) molecules where the V_{H} and V_{L} partner domains are linked via a flexible oligopeptide (Bird et al (1988) Science 242, 423; Huston et al (1988) Proc. Natl. Acad. Sci. USA 85, 5879) and single domain antibodies (dAbs) comprising isolated V domains (Ward et al (1989) Nature 341, 544).

The term "antibody variant" includes any synthetic antibodies, recombinant antibodies or antibody hybrids, such as but not limited to, a single-chain antibody molecule produced by phage-display of immunoglobulin light and/or heavy chain variable and/or constant regions, or other immunointeractive molecule capable of binding to an antigen in an immunoassay format that is known to those skilled in the art.

A general review of the techniques involved in the synthesis of antibody fragments which retain their specific binding sites is to be found in Winter & Milstein (1991) Nature 349, 293-299.

Molecular libraries such as antibody libraries (Clackson et al, 1991, Nature 352, 624-628; Marks et al, 1991, J Mol Biol 222(3): 581-97), peptide libraries (Smith, 1985, Science 228(4705): 1315-7), expressed cDNA libraries (Santi et al (2000) J Mol Biol 296(2): 497-508), libraries on other scaffolds than the antibody framework such as affibodies (Gunneriusson et al, 1999, Appl Environ Microbiol 65(9): 4134-40) or libraries based on aptamers (Kenan et al, 1999, Methods Mol Biol 118, 217-31) may be used as a source from which binding molecules that are specific for a given motif are selected for use in the methods of the invention.

The molecular libraries may be expressed *in vivo* in prokaryotic (Clackson *et al,* 1991, *op. cit*.; Marks *et al,* 1991, *op. cit.*) or eukaryotic cells (Kieke et al, 1999, Proc Natl Acad Sci USA, 96(10):5651-6) or may be expressed *in vitro* without involvement of cells (Hanes & Pluckthun, 1997, Proc Natl Acad Sci USA 94(10):4937-42; He & Taussig, 1997, Nucleic Acids Res 25(24):5132-4; Nemoto et al, 1997, FEBS Lett, 414(2):405-8).

In cases when protein based libraries are used often the genes encoding the libraries of potential binding molecules are packaged in viruses and the potential binding molecule is displayed at the surface of the virus (Clackson *et al,* 1991, *op. cit*.; Marks *et al,* 1991, *op. cit*; Smith, 1985, *op. cit.*).

The most commonly used such system today is filamentous bacteriophage displaying antibody fragments at their surfaces, the antibody fragments being expressed as a fusion to the minor coat protein of the bacteriophage (Clackson *et al,* 1991, *op. cit*.; Marks *et al,* 1991, *op. cit*). However, also other systems for display using other viruses (EP 39578), bacteria (Gunneriusson *et al,* 1999, *op. cit*.; Daugherty et al, 1998, Protein Eng 11(9):825-32; Daugherty et al, 1999, Protein Eng 12(7):613-21), and yeast (Shusta et al, 1999, J Mol Biol 292(5):949-56) have been used.

In addition, display systems have been developed utilising linkage of the polypeptide product to its encoding mRNA in so called ribosome display systems (Hanes & Pluckthun, 1997, *op. cit*.; He & Taussig, 1997, *op. cit*.; Nemoto *et al*, 1997, *op. cit*.), or alternatively linkage of the polypeptide product to the encoding DNA (see US Patent No. 5,856,090 and WO 98/37186).

When potential binding molecules are selected from libraries one or a few selector peptides having defined motifs are usually employed. Amino acid residues that provide structure, decreasing flexibility in the peptide or charged, polar or hydrophobic side chains allowing interaction with the binding molecule may be used in the design of motifs for selector peptides.

For example:
(i) Proline may stabilise a peptide structure as its side chain is bound both to the alpha carbon as well as the nitrogen;
(ii) Phenylalanine, tyrosine and tryptophan have aromatic side chains and are highly hydrophobic, whereas leucine and isoleucine have aliphatic side chains and are also hydrophobic;
(iii) Lysine, arginine and histidine have basic side chains and will be positively charged at neutral pH, whereas aspartate and glutamate have acidic side chains and will be negatively charged at neutral pH;
(iv) Asparagine and glutamine are neutral at neutral pH but contain a amide group which may participate in hydrogen bonds;
(v) Serine, threonine and tyrosine side chains contain hydroxyl groups, which may participate in hydrogen bonds.

Typically, selection of binding agents may involve the use of array technologies and systems to analyse binding to spots corresponding to types of binding molecules.

In an alternative or additional embodiment, the first binding agent(s) is/are immobilised on a surface (*e.g.* on a multiwell plate or array).

The variable heavy (V_{H}) and variable light (V_{L}) domains of the antibody are involved in antigen recognition, a fact first recognised by early protease digestion experiments. Further confirmation was found by "humanisation" of rodent antibodies. Variable domains of rodent origin may be fused to constant domains of human origin such that the resultant antibody retains the antigenic specificity of the rodent parented antibody (Morrison et al (1984) Proc. Natl. Acad. Sci. USA 81, 6851-6855).

That antigenic specificity is conferred by variable domains and is independent of the constant domains is known from experiments involving the bacterial expression of antibody fragments, all containing one or more variable domains. These molecules include Fab-like molecules (Better et al (1988) Science 240, 1041); Fv molecules (Skerra et al (1988) Science 240, 1038); single-chain Fv (ScFv) molecules where the V_{H} and V_{L} partner domains are linked via a flexible oligopeptide (Bird et al (1988) Science 242, 423; Huston et al (1988) Proc. Natl. Acad. Sci. USA 85, 5879) and single domain antibodies (dAbs) comprising isolated V domains (Ward et al (1989) Nature 341, 544). A general review of the techniques involved in the synthesis of antibody fragments which retain their specific binding sites is to be found in Winter & Milstein (1991) Nature 349, 293-299.

By "ScFv molecules" we mean molecules wherein the V_{H} and V_{L} partner domains are linked via a flexible oligopeptide.

The advantages of using antibody fragments, rather than whole antibodies, are several-fold. The smaller size of the fragments may lead to improved pharmacological properties, such as better penetration of solid tissue. Effector functions of whole antibodies, such as complement binding, are removed. Fab, Fv, ScFv and dAb antibody fragments can all be expressed in and secreted from *E. coli,* thus allowing the facile production of large amounts of the said fragments.

Whole antibodies, and F(ab')₂ fragments are "bivalent". By "bivalent" we mean that the said antibodies and F(ab')₂ fragments have two antigen combining sites. In contrast, Fab, Fv, ScFv and dAb fragments are monovalent, having only one antigen combining sites.

The antibodies may be monoclonal or polyclonal. Suitable monoclonal antibodies may be prepared by known techniques, for example those disclosed in "Monoclonal Antibodies: A manual of techniques", H Zola (CRC Press, 1988) and in "Monoclonal Hybridoma Antibodies: Techniques and applications", J G R Hurrell (CRC Press, 1982).

In an alternative or additional embodiment, the first binding agent is immobilised on a surface (*e.g.* on a multiwell plate or array).

Hence, the first binding agent may comprise or consist of an antibody or an antigen-binding fragment thereof. Preferably, the antibody or antigen-binding fragment thereof is a recombinant antibody or antigen-binding fragment thereof. The antibody or antigen-binding fragment thereof may be selected from the group consisting of: scFv, Fab, and a binding domain of an immunoglobulin molecule.

The first binding agent may be immobilised on a surface.

The two or more biomarkers in the test sample may be labelled with a detectable moiety. By a "detectable moiety" we include the meaning that the moiety is one which may be detected and the relative amount and/or location of the moiety (for example, the location on an array) determined.

Suitable detectable moieties are well known in the art.

Thus, the detectable moiety may be a fluorescent and/or luminescent and/or chemiluminescent moiety which, when exposed to specific conditions, may be detected. For example, a fluorescent moiety may need to be exposed to radiation (*i.e.* light) at a specific wavelength and intensity to cause excitation of the fluorescent moiety, thereby enabling it to emit detectable fluorescence at a specific wavelength that may be detected.

Alternatively, the detectable moiety may be an enzyme which is capable of converting a (preferably undetectable) substrate into a detectable product that can be visualised and/or detected. Examples of suitable enzymes are discussed in more detail below in relation to, for example, ELISA assays.

Alternatively, the detectable moiety may be a radioactive atom which is useful in imaging. Suitable radioactive atoms include ^{99m}Tc and ¹²³I for scintigraphic studies. Other readily detectable moieties include, for example, spin labels for magnetic resonance imaging (MRI) such as ¹²³I again, ¹³¹I, ¹¹¹In, ¹⁹F, ¹³C, ¹⁵N, ¹⁷O, gadolinium, manganese or iron. Clearly, the agent to be detected (such as, for example, the one or more biomarkers in the test sample and/or control sample described herein and/or an antibody molecule for use in detecting a selected protein) must have sufficient of the appropriate atomic isotopes in order for the detectable moiety to be readily detectable.

The radio- or other labels may be incorporated into the agents used in the invention (i.e. the proteins present in the samples of the methods of the invention and/or the binding agents used in the invention) in known ways. For example, if the binding moiety is a polypeptide it may be biosynthesised or may be synthesised by chemical amino acid synthesis using suitable amino acid precursors involving, for example, fluorine-19 in place of hydrogen. Labels such as ^{99m}Tc, ¹²³I, ¹⁸⁶Rh, ¹⁸⁸Rh and ¹¹¹In can, for example, be attached *via* cysteine residues in the binding moiety. Yttrium-90 can be attached via a lysine residue. The IODOGEN method (Fraker et al (1978) Biochem. Biophys. Res. Comm. 80, 49-57) can be used to incorporate ¹²³I. Reference ("Monoclonal Antibodies in Immunoscintigraphy", J-F Chatal, CRC Press, 1989) describes other methods in detail. Methods for conjugating other detectable moieties (such as enzymatic, fluorescent, luminescent, chemiluminescent or radioactive moieties) to proteins are well known in the art.

Preferably, the two or more biomarkers in the control sample(s) are labelled with a detectable moiety. The detectable moiety may be selected from the group consisting of: a fluorescent moiety; a luminescent moiety; a chemiluminescent moiety; a radioactive moiety; an enzymatic moiety. However, it is preferred that the detectable moiety is biotin.

In an additional embodiment step (b), (d) and/or step (f) is performed using an assay comprising second binding agents capable of binding to the two or more biomarkers, the second binding agent comprising a detectable moiety. Preferably, the second binding agent comprises or consists of an antibody or an antigen-binding fragment thereof. Preferably, the antibody or antigen-binding fragment thereof is a recombinant antibody or antigen-binding fragment thereof. Most preferably, the antibody or antigen-binding fragment thereof is selected from the group consisting of: scFv, Fab and a binding domain of an immunoglobulin molecule. In an alternative or additional embodiment the detectable moiety is selected from the group consisting of: a fluorescent moiety; a luminescent moiety; a chemiluminescent moiety; a radioactive moiety and an enzymatic moiety. Preferably, the detectable moiety is fluorescent moiety (for example an Alexa Fluor dye, *e.g.* Alexa647).

In an alternative or additional instance, the method of the first aspect of the invention can be carried out by means of an ELISA (Enzyme Linked Immunosorbent Assay). Preferred assays for detecting serum or plasma proteins include enzyme linked immunosorbent assays (ELISA), radioimmunoassay (RIA), immunoradiometric assays (IRMA) and immunoenzymatic assays (IEMA), including sandwich assays using monoclonal and/or polyclonal antibodies. Exemplary sandwich assays are described by David et al in US Patent Nos. 4,376,110 and 4,486,530. Antibody staining of cells on slides may be used in methods well known in cytology laboratory diagnostic tests, as well known to those skilled in the art.

Typically, the assay is an ELISA (Enzyme Linked Immunosorbent Assay) which typically involves the use of enzymes giving a coloured reaction product, usually in solid phase assays. Enzymes such as horseradish peroxidase and phosphatase have been widely employed. A way of amplifying the phosphatase reaction is to use NADP as a substrate to generate NAD which now acts as a coenzyme for a second enzyme system. Pyrophosphatase from *Escherichia coli* provides a good conjugate because the enzyme is not present in tissues, is stable and gives a good reaction colour. Chemi-luminescent systems based on enzymes such as luciferase can also be used.

ELISA methods are well known in the art, for example see The ELISA Guidebook (Methods in Molecular Biology), 2000, Crowther, Humana Press, ISBN-13: 978-0896037281.

Conjugation with the vitamin biotin is frequently used since this can readily be detected by its reaction with enzyme-linked avidin or streptavidin to which it binds with great specificity and affinity.

However, step (b), (d) and/or step (f) is alternatively performed using an array. Arrays *per se* are well known in the art. Typically they are formed of a linear or two-dimensional structure having spaced apart (*i.e.* discrete) regions ("spots"), each having a finite area, formed on the surface of a solid support. An array can also be a bead structure where each bead can be identified by a molecular code or colour code or identified in a continuous flow. Analysis can also be performed sequentially where the sample is passed over a series of spots each adsorbing the class of molecules from the solution. The solid support is typically glass or a polymer, the most commonly used polymers being cellulose, polyacrylamide, nylon, polystyrene, polyvinyl chloride or polypropylene. The solid supports may be in the form of tubes, beads, discs, silicon chips, microplates, polyvinylidene difluoride (PVDF) membrane, nitrocellulose membrane, nylon membrane, other porous membrane, non-porous membrane (e.g. plastic, polymer, perspex, silicon, amongst others), a plurality of polymeric pins, or a plurality of microtitre wells, or any other surface suitable for immobilising proteins, polynucleotides and other suitable molecules and/or conducting an immunoassay. The binding processes are well known in the art and generally consist of cross-linking covalently binding or physically adsorbing a protein molecule, polynucleotide or the like to the solid support. By using well-known techniques, such as contact or non-contact printing, masking or photolithography, the location of each spot can be defined. For reviews see Jenkins, R.E., Pennington, S.R. (2001, Proteomics, 2, 13-29) and Lal et al (2002, Drug Discov Today 15;7(18 Suppl):S143-9).

Typically the array is a microarray. By "microarray" we include the meaning of an array of regions having a density of discrete regions of at least about 100/cm², and preferably at least about 1000/cm². The regions in a microarray have typical dimensions, e.g., diameters, in the range of between about 10-250 µm, and are separated from other regions in the array by about the same distance. The array may also be a macroarray or a nanoarray.

Once suitable binding molecules (discussed above) have been identified and isolated, the skilled person can manufacture an array using methods well known in the art of molecular biology.

Hence, the array may be the array is a bead-based array or a surface-based array. Preferably, the array is selected from the group consisting of: macroarray, microarray and nanoarray.

In an additional embodiment, the method according to the first aspect of the invention comprises:
(i) labelling biomarkers present in the sample with biotin;
(ii) contacting the biotin-labelled proteins with an array comprising a plurality of scFv immobilised at discrete locations on its surface, the scFv having specificity for two or more of the proteins in Table III, including HADH2;
(iii) contacting the immobilised scFv with a streptavidin conjugate comprising a fluorescent dye; and
(iv) detecting the presence of the dye at discrete locations on the array surface
wherein the expression of the dye on the array surface is indicative of the expression of a biomarker from Table A in the sample.

In an alternative embodiment step (b), (d) and/or (f) comprises measuring the expression of a nucleic acid molecule encoding the two or more biomarkers. Preferably the nucleic acid molecule is a cDNA molecule or an mRNA molecule. Most preferably the nucleic acid molecule is an mRNA molecule.

Hence the expression of the two or more biomarker(s) in step (b), (d) and/or (f) may be performed using a method selected from the group consisting of Southern hybridisation, Northern hybridisation, polymerase chain reaction (PCR), reverse transcriptase PCR (RT-PCR), quantitative real-time PCR (qRT-PCR), nanoarray, microarray, macroarray, autoradiography and *in situ* hybridisation. Preferably, the expression of the one or more biomarker(s) in step (b) is determined using a DNA microarray.

In an alternative or additional embodiment, the measuring of the expression of the two or more biomarker(s) in step (b), (d) and/or (f) is performed using two or more binding moieties, each individually capable of binding selectively to a nucleic acid molecule encoding one of the biomarkers identified in Table A.

In a further embodiment, the one or more binding moieties each comprise or consist of a nucleic acid molecule. Thus, the one or more binding moieties may each comprise or consist of DNA, RNA, PNA, LNA, GNA, TNA or PMO. However, it is preferred that the one or more binding moieties each comprise or consist of DNA.

Preferably, the one or more binding moieties are 5 to 100 nucleotides in length. More preferably, the one or more nucleic acid molecules are 15 to 35 nucleotides in length. More preferably still, the binding moiety comprises a detectable moiety.

In an additional embodiment, the detectable moiety is selected from the group consisting of: a fluorescent moiety; a luminescent moiety; a chemiluminescent moiety; a radioactive moiety (for example, a radioactive atom); and an enzymatic moiety. Preferably, the detectable moiety comprises or consists of a radioactive atom. The radioactive atom may be selected from the group consisting of technetium-99m, iodine-123, iodine-125, iodine-131, indium-111, fluorine-19, carbon-13, nitrogen-15, oxygen-17, phosphorus-32, sulphur-35, deuterium, tritium, rhenium-186, rhenium-188 and yttrium-90.

However, the detectable moiety of the binding moiety may be a fluorescent moiety (for example an Alexa Fluor dye, *e.g.* Alexa647).

In an additional embodiment, the sample provided in step (b), (d) and/or (f) is selected from the group consisting of unfractionated blood, plasma, serum, tissue fluid, pancreatic tissue, pancreatic juice, bile and urine. Preferably, the sample provided in step (b), (d) and/or (f) is selected from the group consisting of unfractionated blood, plasma and serum. More preferably, the sample provided in step (b), (d) and/or (f) is plasma. In another preferred embodiment, the sample provided in step (b), (d) and/or (f) is serum.

In an alternative embodiment, the method of the first aspect of the invention is for differentiating body and/or tail pancreatic cancer from head pancreatic cancer comprising or consisting of the steps of:
a) providing a sample to be tested obtained from the individual;
b) determining a biomarker signature of the test sample by measuring the expression in the test sample at least two biomarkers selected from the group defined in Table A (i), (ii), (iv) and/or (vi), including HADH2;
wherein the expression in the test sample of the at least one biomarker selected from the group defined in Table A (i), (ii) and/or (iii) is indicative of the presence of body and/or tail pancreatic cancer, or head pancreatic cancer. The test sample may already have been identified as being a pancreatic cancer sample (for example, using the method described above). Alternatively, the test sample may not have been identified as being a pancreatic cancer sample. Positive and negative control samples can be selected appropriately.

In an additional embodiment, the patient is treated appropriately following identification of a pancreatic cancer. For example, the tumour(s) may be surgically removed (resected), treated with chemotherapy (i.e., anti-neoplastic drugs) and/or treated with radiotherapy. Hence, the present invention includes a method of treating a person having pancreatic cancer, wherein the patient is identified as having pancreatic cancer using the method of the first aspect of the invention.

Also described herein is an array for determining the presence of pancreatic cancer in an individual as defined in the claims herein.

Arrays suitable for use in the methods of the invention are discussed above.

Preferably the two or more binding agents are capable of binding to all of the biomarkers defined in Table A.

A second aspect of the present invention provides the use of two or more biomarkers (including HADH2) selected from the group defined in the first aspect of the invention as a diagnostic marker for determining the presence of pancreatic cancer in an individual *in vitro,* as defined in the claims herein. Preferably, all of the proteins defined in Table III are used as diagnostic markers for determining the presence of pancreatic cancer in an individual.

Also described herein is a kit for determining the presence of pancreatic cancer comprising:
A) one or more first binding agent or an array as described herein; and
B) instructions for performing the method according to the first aspect of the invention.

Also described herein is the use of one or more binding agents/moieties selected from the group defined in the first aspect of the invention for determining the presence of pancreatic cancer in an individual. Preferably, binding agents/moieties for all of the proteins defined in Table III are used as diagnostic markers for determining the presence of pancreatic cancer in an individual.

Preferred, non-limiting examples which embody certain aspects of the invention will now be described, with reference to the following tables and figures:
**Figure 1****.** PCA with samples colored according to diagnosis (red = PC, yellow = BC, blue = NC). The data has been filtered to p = 1e-10 (63 antibodies).
**Figure 2****.** Backward elimination analysis. The Kullback-Leibler (K-L) error after each round of antibody elimination in the first training sets was plotted for **(A)** PC vs NC and **(B)** PC vs BC. **(C)** Boxplot of the AUC values generated from 25-antibody SVM models in ten different pairs of training/test sets.
**Figure 3****.** PCA with samples colored according to tumor localization (red = head tumors, yellow = body and tail tumors, blue = normal controls). The data has been filtered to p = 1e-10 (46 antibodies).
**Figure 4****.** Representative image of a microarray slide with 13 subarrays denoted A1-6 and B1-7, after sample hybridization and scanning. Subarray B4 is enlarged, showing the array lay-out, with 33x31 spots. The arrays consist of three segments separated by printed rows of labeled BSA (row 1, 11, 21, and 31). Each antibody was printed in three replicate spots, one in each segment, distributed to different positions within each segment.
**Figure 5****.** Preprocessing of data shown as PCA plots. A) Raw data colored according to the five rounds (days) of analysis, denoted R1-R5. B) Normalized data showing that clustering based on rounds of analysis has been eliminated.
**Figure 6****.** Pathway analysis on diseases as identified by biomarkers, based on data from the complete set of antibodies, with corresponding p-values and fold changes for PC vs. NC.

### EXAMPLES

### Materials and methods

### Samples

After informed consent, 338 serum samples were collected at five different sites in Spain, from patients with pancreatic cancer (PC, n=156), benign controls (BC, n=152), and from normal controls (NC, n=30) (Table 1). The entire set of samples was labeled at a single occasion, using a previously optimized protocol [32, 33]. Briefly, crude samples were diluted 1:45 in PBS, resulting in an approximate protein concentration of 2 mg/mL, and labeled with a 15:1 molar excess of biotin to protein using 0.6 mM EZ-Link Sulfo-NHS-LC-Biotin (Pierce, Rockford, IL, USA). Unbound biotin was removed by dialysis against PBS for 72 hours. Labeled samples were aliquoted and stored at -20°C.

### Antibodies

In total, 293 human recombinant scFv antibodies directed against 98 known serum antigens and 31 4-6 amino acid motifs (here denoted CIMS 1-31) [34], were used as content of the antibody microarrays (See Table 4 for full list of antibodies used). The antibodies were produced in 15 mL *E. coli* cultures and purified from the periplasm in 300 µL using a MagneHis Protein Purification system (Promega, Madison, WI, USA) and a KingFisher96 robot (Thermo Fisher Scientific, Waltham, MA, USA). The elution buffer was exchanged for PBS using Zeba 96-well desalt spin plates (Pierce). The protein yield was measured using NanoDrop (Thermo Scientific, Wilmington, DE, USA) and the purity was checked using 10% SDS-PAGE (Invitrogen, Carlsbad, CA, USA).

### Antibody microarrays

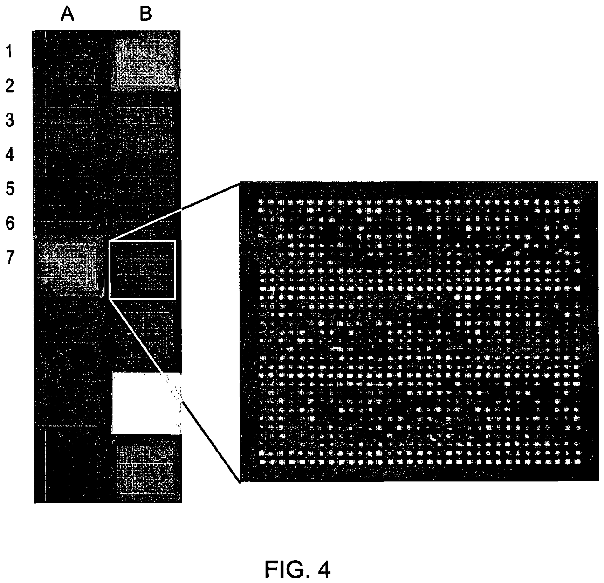

The antibody microarrays were produced on black MaxiSorp slides (NUNC, Roskilde, Denmark) using a non-contact printer (SciFlexarrayer S11, Scienion, Berlin, Germany). Thirteen identical subarrays were printed on each slide, each array consisting of 31x33 spots, 130 µm in diameter, with 200 µm spot-to-spot center distance. Each subarray consisted of three segments, separated by printed rows of labeled BSA (Fig. 4). Each antibody was printed in three replicates, one in each subarray segment, placed in different positions to assure adequate reproducibility. Eight slides, i.e. 104 subarrays, were printed each day for five days. The printing was performed over night and the slides were immediately used for array analysis the following day.

Each slide was mounted in a hybridization gasket (Schott, Jena, Germany) and blocked with PBSMT (1% (w/v) milk, 1% (v/v) Tween-20 in PBS) for one hour. Meantime, aliquots of labeled samples were thawed on ice and diluted 1:10 in PBSMT, in 96-well plates. The slides were washed with PBST (0.05% (v/v) Tween-20 in PBS) four times before the samples were added from the dilution plates. Samples were incubated for two hours on a rocking table, washed four times with PBST, incubated with 1 µg/mL Streptavidin-Alexa in PBSMT for one hour on a rocking table, and again washed four times with PBST. Finally, the slides were dismounted from the hybridization chambers, quickly immersed in dH₂O, and dried under a stream of N₂. The slides were immediately scanned in a confocal microarray scanner (PerkinElmer Life and Analytical Sciences, Wellesley, MA, USA) at 10 µm resolution, using 60% PMT gain and 90% laser power. Signal intensities were quantified using the ScanArray Express software version 4.0 (PerkinElmer Life and Analytical Sciences), using the fixed circle option. Intensities values with local background subtraction were used for data analysis.

### Data pre-processing

An average value of the three replicate spots was used, unless any replicate CV exceeded 15% from the mean value, in case it was ruled out, and the average value of the two remaining replicates was used instead. The average replicate CV was 8.3% (±5.5%). Applying a cut-off CV of 15%, 70% of data values were calculated from all three replicate spots, and the remaining 30% from two replicates.

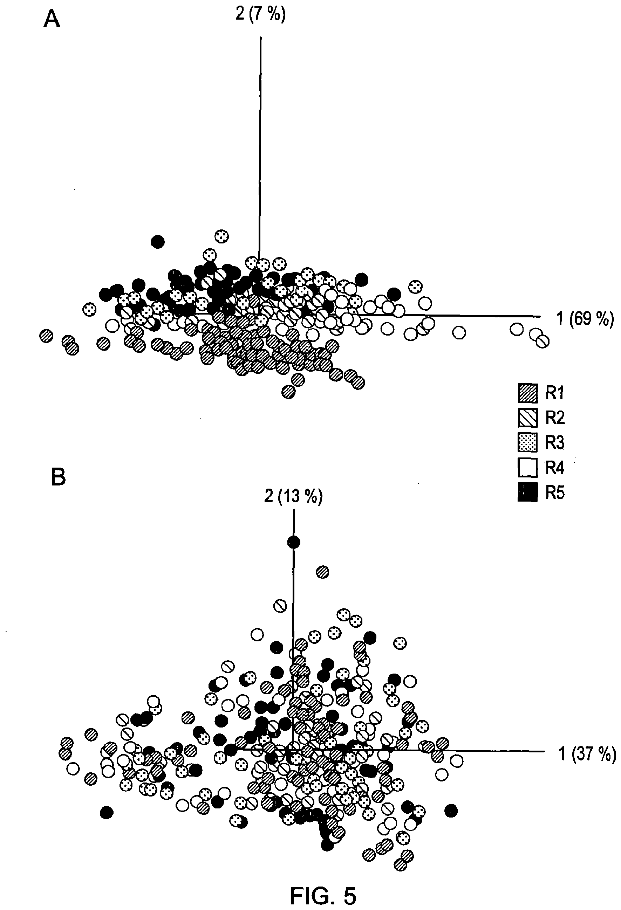

For evaluation of normalization strategies and initial analysis of variance, the data was visualized using 3D principal component analysis (Qlucore, Lund, Sweden). One (chronic pancreatitis) sample was considered to be an outlier (barely any signals were obtained) and was excluded from the data. Principal component analysis on log10 raw data showed that no differences between i) sample subarray positioning on slide, ii) patient gender, iii) patient age, and iv) serum sample collection center, could be found. Minor systematic differences could only be observed between days of analysis (rounds 1-5, likely due to small differences in humidity during array printing, in particular for day 1, see Fig. 4), which was neutralized by normalization. The data was normalized in two steps. First, the differences between rounds (days) of analysis was eliminated using a subtract group mean strategy. The average intensity from each antibody was calculated within each round of analysis, and subtracted from the single values, thus zero centering the data. The global mean signal from each antibody was added to each respective data point to avoid negative values. Second, array-to-array differences (e.g. inherent sample background fluorescence differences (see Fig. 4)) were handled by calculating a scaling factor for each subarray, based on the 20% of antibodies with the lowest CV, as has been previously described [23, 35]. Normalization of data was visualized in PCA plots (Fig. 5).

### Data analysis

Analysis was based on two-group comparisons of PC vs. NC and PC vs. BC using PCA, hierarchical clustering, Student's t-test and fold changes, as well as multigroup ANOVA (PC, NC and CP) (Qlucore). Support Vector Machine (SVM) analysis was performed in R (www.r-project.org) using a linear kernel with the cost of constraints set to one (default value). The data was randomly divided into training and test sets with two thirds of the samples from each group in the training set and the remaining one third of samples in the test set. The backward elimination algorithm was applied using training set data, excluding one antibody at the time and iteratively eliminating the antibody that was removed when the smallest Kullback-Leibler divergence was obtained in the classification analysis, as has been previously described [31]. The last 25 antibodies to be eliminated were used to build a classification model in the training set that was directly applied in the corresponding test set. The area under the ROC-curve (AUC) was used as a measure of the accuracy of the signature in the test set. This procedure was repeated ten times, in ten different, randomly generated pairs of training and test sets. Ultimately, each antibody was given a score based on the order of elimination in the ten training sets. The score was calculated from the average endurance in the elimination process (first antibody to be eliminated = 1, last antibody to be eliminated = 293). Sensitivities (SN) and specificities (SP) were calculated from the optimal threshold of SVM prediction values, here defined as the highest sum of SN+SP. Finally, pathway analyses were performed using MetaCore (Thomson Reuters, New York, NY, USA).

### Results

### Differential protein expression analysis

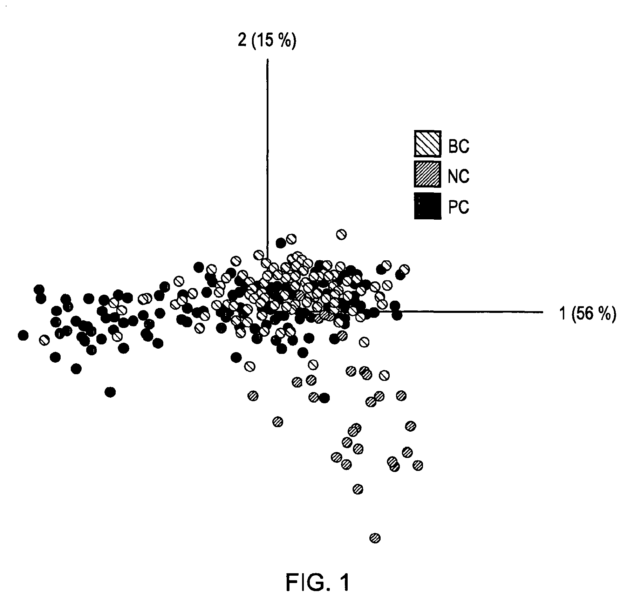

Analysis of variance revealed a large number of antibodies with strong differential signaling patterns between cancer and controls in the serum samples. In fact, multigroup ANOVA showed that 75% of the antibodies displayed a significance level of p<0.001 in the serum data. Principal component analysis demonstrated that the cancer samples were clearly more differentiated from normal than from benign controls (Fig. 1).

For each subgroup comparison, the 25 antibodies displaying the lowest p-values are shown in Table 2. The PC vs. NC comparison revealed strongly up- and down-regulated analytes, while the PC vs. BC analysis identified analytes with more modest fold changes, and which were almost exclusively upregulated in PC vs.BC (Table 2). The analytes presenting the highest level of differential expression were GAK, IL-6, LDL, and MAPK8 (p≤4·10⁻²⁰) for PC vs. NC, while a significantly different set of proteins were identified for PC vs. BC, with Cystatin C, IL-13, and IL-1α displaying the lowest p-values (p≤3·10⁻¹⁰).

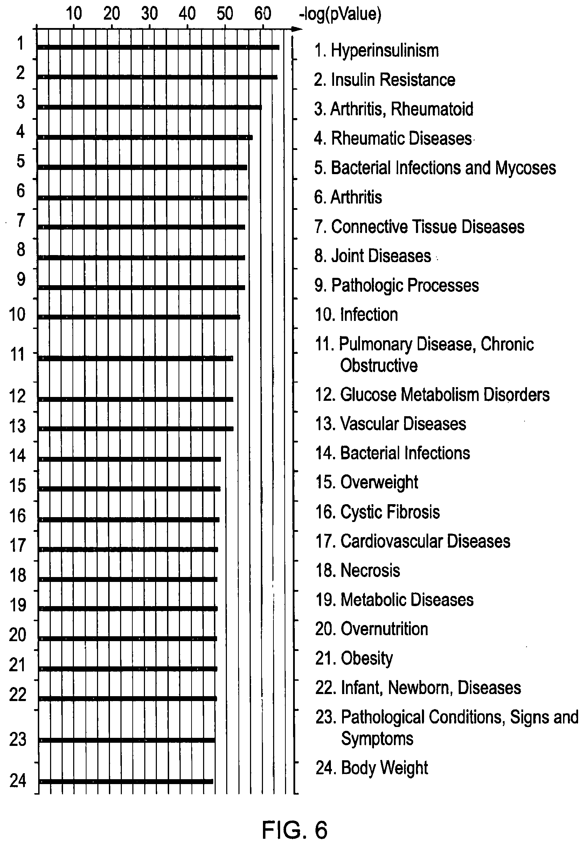

A pathway analysis based on the entire set of antibodies (n=293) with corresponding p-values and fold changes for PC vs. NC, suggested diseases related to insulin production (hyperinsulinism and insulin resistance) as top hits when searching for diseases by biomarkers (Fig. 6). Conditions associated with metabolism, such as glucose metabolism disorders, obesity, and overweight were also significantly related, as well as core biomarker networks of diabetes type I and II, Crohn's disease, hepatitis, autoimmune and infectious conditions, and various types of neoplasms (including pancreatic). The majority of diseases identified by pathway analysis thus has been associated with, or is symptomatically correlated to PC.

### Signatures for classification

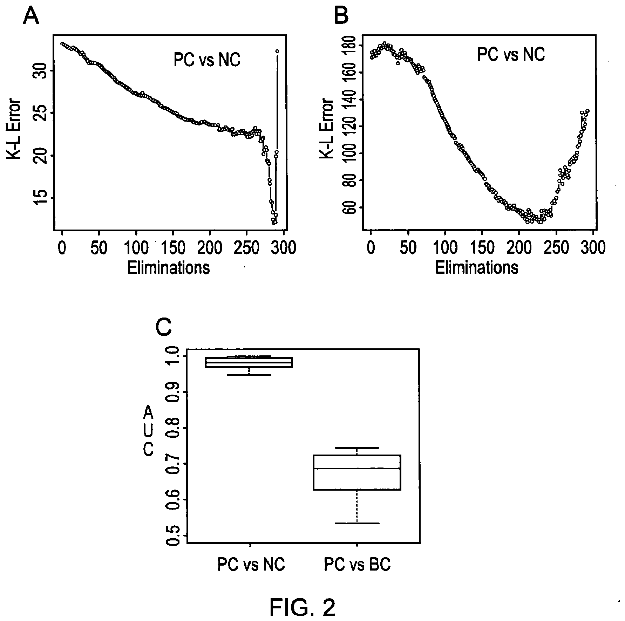

Next, the data was randomly subdivided into training and test sets. The training sets were used to identify the most discriminative combination of antibodies by applying a backward elimination algorithm based on SVM analysis, excluding the antibodies one by one. The classification of PC and NC was highly accurate, as implied by small Kullback-Leibler (K-L) divergences (≤33.2) throughout the elimination process (Fig. 2A). A distinct K-L minimum (12.0) was reached when only seven antibodies were left in the elimination process. This 7-plex protein panel, including IL-6, Cystatin C, IL-8, IL-11, C1 inhibitor, Eotaxin, and HADH2, displayed a sensitivity (SN) and specificity (SP) of 100% and 96%, respectively, in the corresponding test set of previously unseen samples, which demonstrated that a handful of analytes could be combined for a close to perfect classification of PC and NC. In contrast, the K-L values were significantly higher (≤181.3) when PC was compared to BC. Here, the minimum K-L value (50.0) was not as distinct, and implied that a much larger panel of antibodies were needed for optimal differentiation of PC and BC (Fig. 2B). For each subgroup comparison, the procedure was repeated until ten different, randomly generated training sets had been used for backward elimination. The resulting K-L curves were highly similar to those shown in Fig 2, indicating that an average of 67 antibodies would be needed for optimal classification of PC vs. BC (Fig. 2B).

To generate and evaluate signatures of a feasible number of antibodies, we chose the top 25 antibodies from each elimination process and used these to build SVM classification models in the training sets. The AUC values generated by the models in the corresponding test sets were used as a measure of the classification accuracy (Fig. 2C). Each 25-antibody signature generated could predict PC from NC with high accuracy (average AUC 0.98). The sensitivity and specificity of the ten signatures ranged from 90% SN and 85% SP, to 100% SN and SP, with an average SN and SP of 95%. In contrast, PC was more difficult to predict from BC (average AUC 0.67), with 76% SN and 67% SP for the best performing signature.

Finally, each antibody was given a score, corresponding to its average endurance in the elimination processes (Table 3). Within the PC vs. NC analysis, the ten signatures were highly similar. For example, the top antibody, targeting IL-11, had an overall score of 291.4 out of 293 eliminations, being the last antibody to be eliminated 4 out of 10 times. In all, the 25 highest scored antibodies for PC vs. NC represented 20 non-redundant analytes, ranging from cytokines and chemokines (IL-11, IL-6, IL-13, IL-8, TNF-α, and Eotaxin), complement components (C1 inhibitor, C1q, C5, and Factor B), enzymes (HADH2, GAK, and ATP-5B) and more. A highly different set of proteins appeared as top markers for PC vs. BC, with MAPK1, TNFRSF3, UCHL5, IL-4, Apo-A1, Apo-A4, CD40 ligand, KSYK and others, among the top scored analytes (Table 3).

Even though the signatures based on antibody score (Table 3) were different from those derived from p-values (Table 2), a significant overlap was observed, particularly for the PC vs. NC signatures, where 8 out of 25 antibodies (IL-11, IL-6, IL-13, HADH2, LDL, GAK, C1q, and TNF-α) appeared in both signatures. All of the top 25 scored antibodies for PC vs. NC were in fact significantly differentially expressed, even if not all being present on the top 25 p-value list.

### Tumor localization

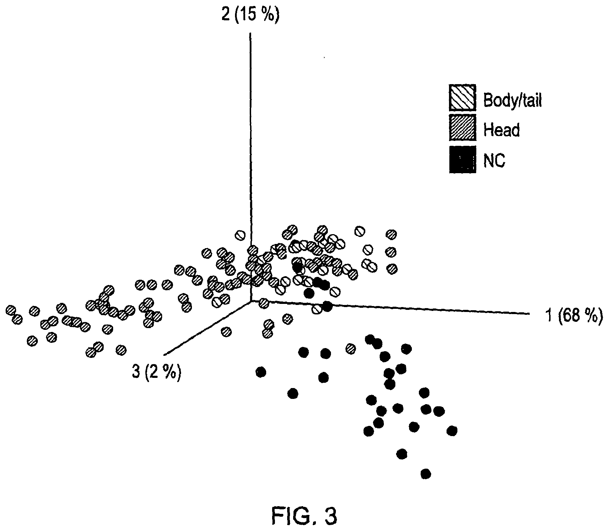

Based on the serum protein profiles, the cancer samples could also be stratified according to tumor origin in the pancreas. Principal component analysis showed that samples from tumors located in the body or the tail of pancreas to some extent clustered closer to the normal controls than what samples from tumors located in the head of pancreas did, and that a separation of the cancer samples based on tumor localization (head vs. body/tail), could be observed (Fig. 3). Differential protein expression analysis revealed an extensive list of analytes with significantly different levels in serum samples from head and body/tail tumors, with 39% of the antibodies displaying p-values < 0.001, and almost exclusively showing upregulated levels in head tumors compared body and tail tumors (Table 5).

### Discussion

This study represents one of the largest multicenter analyses of biomarker panels for predicting pancreatic cancer that has been conducted so far. To the best of our knowledge, this is the most multiplexed analysis of serum analytes in such large pancreatic cancer cohort (>300 samples) using affinity proteomics. Analysis was performed using in-house recombinant antibody microarrays, a platform that has advanced over several years [32, 36], and that now include close to 300 antibodies, stringently selected against a range of targets, mainly of the immunoregulatory proteome. Employing novel protocols for high-throughput production and purification, these antibodies are readily generated in less than a week, and rapidly printed in three replicate spots in the picoliter scale onto planar microarrays. In the current set-up, over a hundred samples can be analyzed in parallel per day and workstation, using only minute volumes (<1 µL) of undiluted serum, enabling large sample cohorts to be analyzed in the course of a few days.

In the present study, 338 serum samples were profiled on these arrays, comparing pancreatic cancer to normal and benign controls. Using highly multiplexed assays such as the current one, a certain level of correlation is likely to appear, particularly when highly interconnected proteins such as those of the immune system, are targeted. Even though the discriminative power of individual antibodies, represented by single p-values, might still be of interest, other statistical approaches may be more accurate for identifying the optimal combination of antibodies [37]. Here, a supervised model based on support vector machine analysis was used. The data was divided into training sets from which biomarker signatures were identified by an iterative backward elimination algorithm, and complementary validation sets, in which the classification power of the signatures was tested.

By adopting this approach, lists of proteins of interest were derived both from differential expression patterns, represented by p-values, and from the backward elimination analysis. The latter showed that only 4 to 10 antibodies would be sufficient for close to perfect discrimination of PC vs. NC, results that are highly encouraging and confirm our previous observations that PC can be readily discriminated from healthy controls by assaying the immunoregulatory proteome [23, 24]. However, when PC was compared to BC (mainly chronic pancreatitis), as many as 67 antibodies appeared to be required for optimal classification power. This can partly be explained by the type of proteins measured; the immunoregulatory analytes are not likely to show single-handed disease specificity, as the immune system is highly affected in any condition. Indeed, the pathway analysis conducted in this study, confirmed the highly similar systemic impact of PC and a range of other conditions, such as hyperinsulinism, insulin resistance and metabolic diseases, as well as autoimmunity and infections, again demonstrating the challenge in distinguishing PC from symptomatically related benign conditions. Identifying a relevant immunosignature for PC is thus an act of balance. A small panel is desirable since each binder adds to the cost and complexity of the assay, still the signature needs to be large enough to constitute a sensitive and specific fingerprint of the disease. At this stage of discovery, we reasoned that 25 analytes would be a feasible starting point, and follow-up studies will tell whether these signatures can be condensed to even smaller biomarker panels, while still displaying the sensitivity and specificity required for a diagnostic immunoassay.

Although adopting two highly different strategies for signature identification, there was still a large overlap between the signatures derived from the different analyses, i.e. the markers that obtained the highest overall backward elimination scores were generally also highly differentially expressed. For example, IL-11, IL-6, C1 inhibitor, IL-13, HADH2, LDL, GAK, C1q, and TNF-α appeared in both signatures, and thus demonstrated both low p-values and high backward elimination scores for PC vs. NC. For PC vs. BC the two signatures overlapped by C5, Apo-A4, BTK, TGF-β1, MCP-1, and UPF3B. Of note, a number of markers showed potential for PC both when compared to NC and to BC. Several of these, including C1 inhibitor, C5, Factor B, IL-13, MCP-1, and TNF-α, have been associated with PC in previous studies by us [23, 24] and others [38-40], while HADH2, an acetyl CoA dehydrogenase, has to the best of our knowledge so far not been reported for PC. In addition, other proteins that have not previously been measured by us also showed potential for PC differentiation. For example, GAK, a serine/threonine kinase was heavily down-regulated in PC vs. NC, and also appeared in the backward elimination signatures for PC vs. NC. In addition TNFRSF3 (TNF-β receptor), and UPF3B, an mRNA regulator protein, were included in both the p-value and antibody score signatures for PC vs. BC. Finally, MAPK1 (ERK2), a kinase of the MAPK/ERK signaling pathway which has shown to be deregulated in PC [41], was the highest scored protein in the backward elimination signatures for PC vs. BC.

The serum samples were also stratified on the basis of tumor location in the pancreas, which to the best of our knowledge has not previously been done with proteomics. It was shown that the samples to a certain extent could be separated based on tumor origin, and that the predominant part of samples from patients with tail and body tumors clustered slightly closer to the normal controls than what serum from patients with pancreatic head tumors did. These results thus demonstrated discrepancies in the systemic impact of the cancer based on its origin in the pancreas. The vast number of serum immunoregulatory proteins found to be upregulated in samples from head tumors compared to samples from body/tail tumors, may reflect a more profound systemic impact from tumors located in the pancreatic head, often invading the surrounding mesenteric blood vessels connecting pancreas to the duodenum [3]. These findings thus suggest that serum profiling on antibody microarrays could be applied to distinguish between head and body/tail pancreatic tumors, which potentially could aid in the decision of tumor treatment.

Despite being the 4th most lethal cancer, the incidence of PC is low, ∼11 per 100 000 individuals in the US [42]. From a health-economic perpective, the low incidence makes it difficult to motivate screening for PC in the general population. Based on sensitivities and specificities previously presented by us [24], a recent study however confirms the cost-effectiveness of screening high-risk individuals for PC [43]. Risk factors for PC include not only pancreatitis and benign neoplasms, but also e.g. familial pancreatic cancer, hereditary pancreatitis, BRCA mutations, Peutz-Jeghers syndrome, diabetes mellitus, and *Helicobacter pylori* infection [44]. The next step will be to explore the signatures identified in the present study, for PC diagnosis among individuals at an increased risk of PC, representing a relevant target population for a diagnostic PC immunoassay.

In conclusion, this extensively multiplexed, multicenter study which revealed immunosignatures associated with pancreatic cancer, displaying sensitivities specificities in the 90-100% range, clearly demonstrated the applicability for PC diagnosis, and also indicated the potential of recombinant antibody microarrays for stratifying serum samples based on tumor location in the pancreas.

### References

[1] Siegel R, Naishadham D, Jemal A. Cancer statistics, 2012. CA: a cancer journal for clinicians. 2012;62:10-29.
[2] Yachida S, Jones S, Bozic I, Antal T, Leary R, Fu B, et al. Distant metastasis occurs late during the genetic evolution of pancreatic cancer. Nature. 2010;467:1114-7.
[3] Hidalgo M. Pancreatic cancer. The New England journal of medicine. 2010;362:1605-17.
[4] Conlon KC, Klimstra DS, Brennan MF. Long-term survival after curative resection for pancreatic ductal adenocarcinoma. Clinicopathologic analysis of 5-year survivors. Annals of surgery. 1996;223:273-9.
[5] Sohn TA, Yeo CJ, Cameron JL, Koniaris L, Kaushal S, Abrams RA, et al. Resected adenocarcinoma of the pancreas-616 patients: results, outcomes, and prognostic indicators. Journal of gastrointestinal surgery : official journal of the Society for Surgery of the Alimentary Tract. 2000;4:567-79.
[6] Furukawa H, Okada S, Saisho H, Ariyama J, Karasawa E, Nakaizumi A, et al. Clinicopathologic features of small pancreatic adenocarcinoma. A collective study. Cancer. 1996;78:986-90.
[7] Shimizu Y, Yasui K, Matsueda K, Yanagisawa A, Yamao K. Small carcinoma of the pancreas is curable: new computed tomography finding, pathological study and postoperative results from a single institute. Journal of gastroenterology and hepatology. 2005;20: 1591-4.
[8] Ishikawa O, Ohigashi H, Imaoka S, Nakaizumi A, Uehara H, Kitamura T, et al. Minute carcinoma of the pancreas measuring 1 cm or less in diameter--collective review of Japanese case reports. Hepato-gastroenterology. 1999;46:8-15.
[9] Gangi S, Fletcher JG, Nathan MA, Christensen JA, Harmsen WS, Crownhart BS, et al. Time interval between abnormalities seen on CT and the clinical diagnosis of pancreatic cancer: retrospective review of CT scans obtained before diagnosis. AJR American journal of roentgenology. 2004;182:897-903.
[10] Pelaez-Luna M, Takahashi N, Fletcher JG, Chari ST. Resectability of presymptomatic pancreatic cancer and its relationship to onset of diabetes: a retrospective review of CT scans and fasting glucose values prior to diagnosis. The American journal of gastroenterology. 2007;102:2157-63.
[11] Locker GY, Hamilton S, Harris J, Jessup JM, Kemeny N, Macdonald JS, et al. ASCO 2006 update of recommendations for the use of tumor markers in gastrointestinal cancer. Journal of clinical oncology : official journal of the American Society of Clinical Oncology. 2006;24:5313-27.
[12] Koopmann J, Rosenzweig CN, Zhang Z, Canto MI, Brown DA, Hunter M, et al. Serum markers in patients with resectable pancreatic adenocarcinoma: macrophage inhibitory cytokine 1 versus CA19-9. Clinical cancer research : an official journal of the American Association for Cancer Research. 2006;12:442-6.
[13] Brand RE, Nolen BM, Zeh HJ, Allen PJ, Eloubeidi MA, Goldberg M, et al. Serum biomarker panels for the detection of pancreatic cancer. Clinical cancer research : an official journal of the American Association for Cancer Research. 2011;17:805-16.
[14] Bunger S, Laubert T, Roblick UJ, Habermann JK. Serum biomarkers for improved diagnostic of pancreatic cancer: a current overview. Journal of cancer research and clinical oncology. 2011;137:375-89.
[15] Jiang JT, Wu CP, Deng HF, Lu MY, Wu J, Zhang HY, et al. Serum level of TSGF, CA242 and CA19-9 in pancreatic cancer. World journal of gastroenterology : WJG. 2004; 10: 1675-7.
[16] Duraker N, Hot S, Polat Y, Hobek A, Gender N, Urhan N. CEA, CA 19-9, and CA 125 in the differential diagnosis of benign and malignant pancreatic diseases with or without jaundice. Journal of surgical oncology. 2007;95:142-7.
[17] Ni XG, Bai XF, Mao YL, Shao YF, Wu JX, Shan Y, et al. The clinical value of serum CEA, CA19-9, and CA242 in the diagnosis and prognosis of pancreatic cancer. European journal of surgical oncology : the journal of the European Society of Surgical Oncology and the British Association of Surgical Oncology. 2005;31:164-9.
[18] Faca VM, Song KS, Wang H, Zhang Q, Krasnoselsky AL, Newcomb LF, et al. A mouse to human search for plasma proteome changes associated with pancreatic tumor development. PLoS medicine. 2008;5:e123.
[19] Firpo MA, Gay DZ, Granger SR, Scaife CL, DiSario JA, Boucher KM, et al. Improved diagnosis of pancreatic adenocarcinoma using haptoglobin and serum amyloid A in a panel screen. World journal of surgery. 2009;33:716-22.
[20] Orchekowski R, Hamelinck D, Li L, Gliwa E, vanBrocklin M, Marrero JA, et al. Antibody microarray profiling reveals individual and combined serum proteins associated with pancreatic cancer. Cancer research. 2005;65:11193-202.
[21] Coussens LM, Werb Z. Inflammation and cancer. Nature. 2002;420:860-7.
[22] Carlsson A, Wuttge DM, Ingvarsson J, Bengtsson AA, Sturfelt G, Borrebaeck CA, et al. Serum protein profiling of systemic lupus erythematosus and systemic sclerosis using recombinant antibody microarrays. Molecular & cellular proteomics : MCP. 2011;10:M110 005033.
[23] Ingvarsson J, Wingren C, Carlsson A, Ellmark P, Wahren B, Engstrom G, et al. Detection of pancreatic cancer using antibody microarray-based serum protein profiling. Proteomics. 2008;8:2211-9.
[24] Wingren C, Sandstrom A, Segersvard R, Carlsson A, Andersson R, Lohr M, et al. Identification of serum biomarker signatures associated with pancreatic cancer. Cancer research. 2012;72:2481-90.
[25] Anderson NL, Anderson NG. The human plasma proteome: history, character, and diagnostic prospects. Molecular & cellular proteomics : MCP. 2002;1:845-67.
[26] Zhang H, Liu AY, Loriaux P, Wollscheid B, Zhou Y, Watts JD, et al. Mass spectrometric detection of tissue proteins in plasma. Molecular & cellular proteomics : MCP. 2007;6:64-71.
[27] Surinova S, Schiess R, Huttenhain R, Cerciello F, Wollscheid B, Aebersold R. On the development of plasma protein biomarkers. Journal of proteome research. 2011; 10:5-16.
[28] Haab BB, Geierstanger BH, Michailidis G, Vitzthum F, Forrester S, Okon R, et al. Immunoassay and antibody microarray analysis of the HUPO Plasma Proteome Project reference specimens: systematic variation between sample types and calibration of mass spectrometry data. Proteomics. 2005;5:3278-91.
[29] Alonzo TA, Pepe MS, Moskowitz CS. Sample size calculations for comparative studies of medical tests for detecting presence of disease. Statistics in medicine. 2002;21 :835-52.
[30] Borrebaeck CA, Wingren C. Design of high-density antibody microarrays for disease proteomics: key technological issues. Journal of proteomics. 2009;72:928-35.
[31] Carlsson A, Wingren C, Kristensson M, Rose C, Ferno M, Olsson H, et al. Molecular serum portraits in patients with primary breast cancer predict the development of distant metastases. Proceedings of the National Academy of Sciences of the United States of America. 2011;108:14252-7.
[32] Wingren C, Ingvarsson J, Dexlin L, Szul D, Borrebaeck CA. Design of recombinant antibody microarrays for complex proteome analysis: choice of sample labeling-tag and solid support. Proteomics. 2007;7:3055-65.
[33] Carlsson A, Persson O, Ingvarsson J, Widegren B, Salford L, Borrebaeck CA, et al. Plasma proteome profiling reveals biomarker patterns associated with prognosis and therapy selection in glioblastoma multiforme patients. Proteomics Clinical applications. 2010;4:591-602.
[34] Olsson N, Wallin S, James P, Borrebaeck CA, Wingren C. Epitope-specificity of recombinant antibodies reveals promiscuous peptide-binding properties. Protein science : a publication of the Protein Society. 2012;21:1897-910.
[35] Carlsson A, Wingren C, Ingvarsson J, Ellmark P, Baldertorp B, Ferno M, et al. Serum proteome profiling of metastatic breast cancer using recombinant antibody microarrays. Eur J Cancer. 2008;44:472-80.
[36] Ingvarsson J, Larsson A, Sjoholm AG, Truedsson L, Jansson B, Borrebaeck CA, et al. Design of recombinant antibody microarrays for serum protein profiling: targeting of complement proteins. Journal of proteome research. 2007;6:3527-36.
[37] Quackenbush J. Computational analysis of microarray data. Nature reviews Genetics. 2001;2:418-27.
[38] Ma Y, Hwang RF, Logsdon CD, Ullrich SE. Dynamic mast cell-stromal cell interactions promote growth of pancreatic cancer. Cancer research. 2013;73:3927-37.
[39] Kudo-Saito C, Shirako H, Ohike M, Tsukamoto N, Kawakami Y. CCL2 is critical for immunosuppression to promote cancer metastasis. Clinical & experimental metastasis. 2013;30:393-405.
[40] McDade TP, Perugini RA, Vittimberga FJ, Jr., Carrigan RC, Callery MP. Salicylates inhibit NF-kappaB activation and enhance TNF-alpha-induced apoptosis in human pancreatic cancer cells. The Journal of surgical research. 1999;83:56-61.
[41] Arlt A, Muerkoster SS, Schafer H. Targeting apoptosis pathways in pancreatic cancer. Cancer letters. 2013;332:346-58.
[42] Shaib YH, Davila JA, EI-Serag HB. The epidemiology of pancreatic cancer in the United States: changes below the surface. Alimentary pharmacology & therapeutics. 2006;24:87-94.
[43] Ghatnekar O, Andersson R, Svensson M, Persson U, Ringdahl U, Zeilon P, et al. Modelling the benefits of early diagnosis of pancreatic cancer using a biomarker signature. International journal of cancer Journal international du cancer. 2013.
[44] Konstantinou F, Syrigos KN, Saif MW. Pancreatic cancer: what about screening and detection? JOP : Journal of the pancreas. 2013;14:312-5.

**Table 1. Serum sample demographics**

| ***Diagnosis*** | ***Subgroup*** | ***Gender (M*/*F)*** | ***Age (mean ± sd)*** | ***No of samples*** |
|---|---|---|---|---|
| Pancreatic cancer (PC) | | 92/64 | 66 ± 13 | **156** |
| | Body | | | 16 |
| | Head | | | 97 |
| | Tail | | | 10 |
| | Other | | | 16 |
| | Unspecified | | | 17 |
| Benign controls (BC) | | 117/35 | 52 ± 14 | **152** |
| | Acute pancreatitis | | | 33 |
| | Chronic pancreatitis | | | 110 |
| | Langerhan neoplasm | | | 3 |
| | Pancreatic neoplams | | | 6 |
| Normal controls (NC) | | 20/10 | 62 ± 14 | **30** |
| **Total** | | | | **338** |

**Table 2. Top 25 antibodies based on significance by differential protein expression analysis. Shown within brackets is the individual antibody clone suffix (for analytes targeted by multiple antibody clones). FC = Fold change**

| **PC-NC-BC ANOVA** | | **PC-NC t-test** | | | **PC-BC t-test** | | |
|---|---|---|---|---|---|---|---|
| *Antibody* | *p-value* | *Antibody* | *p-value* | *FC* | *Antibody* | *p-value* | *FC* |
| GAK (3) | *4.14E-48* | GAK (3) | *3.44E-30* | *0.68* | Cystatin C (3) | *3.80E-11* | *1.08* |
| IL-6 (7) | *4.00E-43* | IL-6 (7) | *1.11E-27* | *0.71* | IL-13 (3) | *1.09E-10* | *1.13* |
| GAK (2) | *3.25E-36* | GAK (1) | *3.25E-26* | *0.77* | IL-1α (1) | *3.08E-10* | *1.11* |
| IL-11 (2) | *1.78E-32* | LDL (2) | *1.15E-22* | *0.62* | Surface ag X | *5.57E-09* | *1.10* |
| LDL (2) | *6.09E-32* | MAPK8 (1) | *4.24E-20* | *0.81* | BTK (2) | *6.06E-09* | *1.05* |
| TNF-α (3) | *1.89E-31* | IL-11 (2) | *4.56E-18* | *0.69* | Cystatin C (4) | *6.60E-09* | *1.05* |
| Procathepsin W | *4.88E-27* | TNF-α (3) | *2.93E-16* | *0.76* | CIMS (26) | *6.79E-09* | *1.10* |
| IL-13 (3) | *8.15E-27* | HADH2 (3) | *2.49E-15* | *0.89* | CD40 (1) | *1.02E-08* | *1.09* |
| MAPK8 (1) | *3.02E-22* | Procathepsin W | *3.21E-14* | *0.79* | TNFRSF3 (2) | *1.29E-08* | *1.05* |
| IL-1α (1) | *5.83E-20* | TNFRSF3 (1) | *5.08E-13* | *0.91* | ORP-3 (2) | *2.16E-08* | *1.07* |
| IL-13 (2) | *3.36E-19* | VEGF (3) | *1.52E-12* | *1.24* | Apo-A4 (3) | *2.66E-08* | *1.07* |
| TNFRSF3 (1) | *8.51E-16* | IL-1ra (1) | *3.24E-12* | *1.16* | UPF3B (2) | *2.84E-08* | *1.04* |
| IL-18 (2) | *5.33E-15* | C1 inh (1) | *1.20E-11* | *1.22* | MUC-1 (1) | *3.49E-08* | *1.07* |
| IL-1ra (1) | *9.67E-15* | IL-13 (3) | *2.73E-11* | *0.77* | TNF-α (3) | *4.31E-08* | *1.08* |
| HADH2 (3) | *1.06E-14* | C1q | *4.12E-11* | *1.11* | CIMS (16) | *4.67E-08* | *1.08* |
| CD40 (1) | *1.48E-14* | IL-16 (3) | *5.19E-11* | *1.15* | ATP-5B (1) | *5.68E-08* | *1.06* |
| Cystatin C (4) | *2.36E-14* | VEGF (1) | *5.43E-11* | *1.19* | CIMS (12) | *6.00E-08* | *1.06* |
| IL-4 (3) | *3.12E-14* | CD40L | *6.21E-11* | *1.13* | IL-13 (1) | *6.31E-08* | *1.08* |
| CIMS (18) | *6.91E-14* | IL-4 (3) | *8.82E-11* | *1.24* | MCP-1 (4) | *9.64E-08* | *1.03* |
| CIMS (16) | *7.21E-14* | Sialyl Lewis x | *9.47E-11* | *1.10* | CIMS (1) | *1.14E-07* | *1.08* |
| VEGF (3) | *8.52E-14* | IL-18 (2) | *1.12E-10* | *1.20* | CD40 (3) | *1.28E-07* | *1.06* |
| FASN (3) | *1.18E-13* | CIMS (18) | *1.43E-10* | *1.16* | Procathepsin W | *1.43E-07* | *1.07* |
| TGF-β1 (2) | *2.59E-13* | CIMS (23) | *2.64E-10* | *1.13* | Digoxin | *1.55E-07* | *1.08* |
| CIMS (26) | *2.61E-13* | MCP-1 (2) | *3.08E-10* | *1.16* | TGF-β1 (2) | *1.88E-07* | *1.09* |
| CIMS (25) | *6.47E-13* | CIMS (25) | *3.44E-10* | *1.24* | CIMS (24) | *3.32E-07* | *1.08* |

**Table 3. Top 25 antibodies based on antibody score from ten backward elimination iterations. Shown within brackets is the individual antibody clone suffix (for analytes targeted by multiple antibody clones).**

| **PC vs NC** | ***Score*** | **PC vs BC** | ***Score*** |
|---|---|---|---|
| IL-11 (2) | *291.4* | MAPK1 (3) | *276.5* |
| IL-6 (7) | *288.1* | C5 (2) | *273* |
| Cystatin C (1) | *286.9* | TNFRSF3 (1) | *265.5* |
| C1 inh (3) | *279.2* | TNFRSF3 (2) | *260.9* |
| Angiomotin (1) | *276* | UCHL5 | *259.6* |
| IL-13 (2) | *272.9* | IL-4 (3) | *258.7* |
| IL-13 (3) | *270.7* | Factor B (3) | *258* |
| CD40 (1) | *270.6* | Apo-A4 (3) | *257.5* |
| HADH2 (3) | *270.4* | KSYK-1 | *255.2* |
| HADH2 (4) | *269.7* | Sox11A | *253.1* |
| C1 inh. (4) | *269.4* | CD40L | *252.2* |
| C1 inh. (2) | *269.2* | Apo-A1 (1) | *251.4* |
| LDL (2) | *268.1* | CIMS (13) | *250.1* |
| GAK (3) | *268* | BTK (2) | *246.1* |
| C3 (1) | *266.1* | GM-CSF (5) | *245* |
| CIMS (5) | *264.3* | TGF-β1 (2) | *239.5* |
| C1q | *261.1* | PTP-1B (2) | *237.2* |
| CD40 (4) | *259.6* | MCP-1 (7) | *235.1* |
| IL-8 (2) | *259.4* | UPF3B (1) | *232.5* |
| C5 (2) | *258.5* | C1 inh. (4) | *228.3* |
| ATP-5B (3) | *257.1* | Sialyl Lewis x | *227.6* |
| Factor B (4) | *256.2* | IL-3 (1) | *225.8* |
| CIMS (10) | *253.6* | IL-9 (2) | *224.2* |
| TNF-α (3) | *253.5* | HADH2 (2) | *222.7* |
| Eotaxin (3) | *248.4* | IL-4 (4) | *222.4* |

**Table 4. Antigens targeted on the antibody microarray**

| *Protein* | *Full name* | *No of antibody clones* |
|---|---|---|
| Angiomotin | Angiomotin | 2 |
| Apo-A1 | Apolipoprotein A1 | 3 |
| Apo-A4 | Apolipoprotein A4 | 3 |
| ATP-5B | ATP synthase subunit beta, mitochondrial | 3 |
| β-galactosidese | Beta-galactosidase | 1 |
| BTK | Tyrosine-protein kinase BTK | 4 |
| C1 inhibitor | Plasma protease C1 inhibitor | 4 |
| C1q | Complement C1q | 1 |
| C1s | Complement C1s | 1 |
| C3 | Complement C3 | 6 |
| C4 | Complement C4 | 4 |
| C5 | Complement C5 | 3 |
| CD40 | CD40 protein | 4 |
| CD40L | CD40 ligand | 1 |
| CDK-2 | Cyclin-dependent kinase 2 | 2 |
| CHX10 | Visual system homeobox 2 | 3 |
| CT17 | Cholera toxin subunit B | 1 |
| Cystatin C | Cystatin C | 4 |
| Digoxin | Digoxin | 1 |
| DUSP9 | Dual specificity protein phosphatase 9 | 1 |
| EGFR | Epidermal growth factor receptor (Cetuximab (Erbltux) antibody) | 1 |
| Eotaxin | Eotaxin | 3 |
| Factor B | Complement factor B | 4 |
| FASN | FASN protein | 4 |
| GAK | GAK protein | 3 |
| GLP-1 | Glucagon-like peptide-1 | 1 |
| GLP-1R | Glucagon-like peptide 1 receptor | 1 |
| GM-CSF | Granulocyte-macrophage colony-stimulating factor | 6 |
| HADH2 | HADH2 protein | 4 |
| Her2/ErbB-2 | Receptor tyrosine-protein kinase erbB-2 | 4 |
| HLA-DR/DP | HLA-DR/DP | 1 |
| ICAM-1 | Intercellular adhesion molecule 1 | 1 |
| IFN-γ | Interferon gamma | 3 |
| IgM | Immunoglobulin M | 5 |
| IL-10 | Interleukin-10 | 3 |
| IL-11 | Intereukin-11 | 3 |
| IL-12 | Interleukin-12 | 4 |
| IL-13 | Inlerleultin-13 | 3 |
| IL-16 | Interleukin-16 | 3 |
| IL-18 | Interleukin-18 | 3 |
| IL-1α | Interleukin-1 alpha | 3 |
| IL-1β | Interleukin-1 beta | 3 |
| IL-1ra | Interleukin-1 receptor antagonist protein | 3 |
| IL-2 | Interleukin-2 | 3 |
| IL-3 | Interleukin-3 | 3 |
| IL-4 | Intereukin-4 | 4 |
| IL-5 | Interleukin-5 | 3 |
| IL-6 | Interleukin-6 | 8 |
| IL-7 | Interleukin-7 | 2 |
| IL-3 | Interleukin-8 | 3 |
| IL-9 | Interleukin-9 | 3 |
| Integrin α-10 | Integrin alpha-10 | 1 |
| Integrin α-11 | Integrin alpha-11 | 1 |
| JAK3 | Tyrosine-protein kinase JAK3 | 1 |
| Keratin 19 | Keratin, type I cytoskeletal 19 | 3 |
| KSYK | Tyrosine-protein kinase SYK | 2 |
| LDL | Apolipoprotein B-100 | 2 |
| Leptin | Leptin | 1 |
| Lewis x | Lewis x | 2 |
| Lewis y | Lewis y | 1 |
| Lumican | Lumican | 1 |
| MAPK1 | Mitogen-activated protein kinase 1 | 4 |
| MAPK8 | Mitogen-activated protein kinase 8 | 3 |
| MATK | Megakaryocyte-associated tyrosine-protein kinase | 3 |
| MCP-1 | C-C motif chemokine 2 | 9 |
| MCP-3 | C-C motif chemokine 7 | 3 |
| MCP-4 | C-C motif chemokine 13 | 3 |
| MUC-1 | Mucin-1 | 6 |
| Myomesin-2 | Myomesin-2 | 2 |
| ORP-3 | Oxysterol-binding protein-retaled protein 3 | 2 |
| Osteopontin | Osteopontin | 3 |
| P85A | Phosphatidylinositol 3-kinase regulatory subunit alpha | 3 |
| PKB gamma | RAC-gamma serine/threonine-protein kinase | 2 |
| Procathepsin W | Cathepsin W | 1 |
| Properdin | Properdin | 1 |
| PSA | Prostate-specific antigen | 1 |
| PTK-6 | Protein-tyrosine kinase 6 | 1 |
| PTP-1B | Tyrosine-protein phosphatase non-receptor type 1 | 3 |
| RANTES | C-C motif chemokine 5 | 3 |
| RPS6KA2 | Ribosomal protein S6 kinase alpha-2 | 3 |
| Sialyl Lewis x | Sialyl Lewis x | 1 |
| Sox11A | Transcription factor SOX-11 | 1 |
| STAP2 | Signal-transducing adaptor protein 2 | 4 |
| STAT1 | Signal transducer and activator of transcription 1-alpha/beta | 2 |
| Surface Ag X | Surface Ag X | 1 |
| TBC1D9 | TBC1 domain family member 9 | 3 |
| TENS4 | Tensin-4 | 1 |
| TGF-β1 | Transforming growth factor beta-1 | 3 |
| TM peptide | Transmembrane peptide | 1 |
| TNF-α | Tumor necrosis factor | 3 |
| TNF-β | Lymphotoxin-alpha | 4 |
| TNFRSF14 | Tumor necrosis factor receptor superfamily member 14 | 2 |
| TNFRSF3 | Tumor necrosis factor receptor superfamily member 3 | 3 |
| UBC9 | SUMO-conjugating enzyme UBC9 | 3 |
| UBE2C | Ubiquitin-conjugating enzyme E2 C | 2 |
| UCHL5 | Ubiquitin carboxyl-terminal hydrolase isozyme L5 | 1 |
| UPF3B | Regulator of nonsense transcripts 3B | 2 |
| VEGF | Vascular endothelial growth factor | 4 |

**Table 5. Differential protein expression analysis of serum samples drawn from patients with differently located pancreatic tumors. Results are shown for body+tail tumors vs. head tumors, for the top 40 antibodies (p<5·10⁻⁵). FC = Fold change.**

| *Antibody* | *p-val* | *FC* |
|---|---|---|
| IL-1α (2) | 4.32E-07 | 0.874447 |
| CIMS (16) | 1.28E-06 | 0.874676 |
| VEGF (1) | 1.47E-06 | 0.870962 |
| TNF-β (2) | 1.57E-06 | 0.872955 |
| IL-11 (2) | 2.15E-06 | 0.833144 |
| CIMS (18) | 3.09E-06 | 0.891206 |
| CD40L | 3.89E-06 | 0.917468 |
| IL-3 (3) | 4.19E-06 | 0.87497 |
| CIMS (30) | 4.88E-06 | 0.919455 |
| IL-6 (1) | 7.01E-06 | 0.873484 |
| HLA-DR/DP | 8.10E-06 | 0.879348 |
| IL-2 (3) | 8.34E-06 | 0.845869 |
| Angiomotin (2) | 8.45E-06 | 0.892806 |
| Integrin α-10 | 9.07E-06 | 0.864779 |
| IL-18 (3) | 9.40E-06 | 0.899675 |
| Sox11A | 1.05E-05 | 0.898637 |
| IL-7 (1) | 1.18E-05 | 0.892816 |
| MCP-1 (3) | 1.20E-05 | 0.885187 |
| Surface ag X | 1.20E-05 | 0.865919 |
| IL-9 (1) | 1.21E-05 | 0.872639 |
| CIMS (20) | 1.36E-05 | 0.873286 |
| IL-12 (3) | 1.67E-05 | 0.872587 |
| Lewis x (1) | 1.77E-05 | 0.898973 |
| IgM (3) | 1.91E-05 | 0.893885 |
| IL-7 (2) | 1.98E-05 | 0.893886 |
| CIMS (25) | 2.25E-05 | 0.851688 |
| CIMS (2) | 2.30E-05 | 0.88583 |
| CIMS (6) | 2.41E-05 | 0.858863 |
| IL-16 (3) | 2.57E-05 | 0.905756 |
| GLP-1 | 2.78E-05 | 0.890237 |
| CHX10 (3) | 2.83E-05 | 0.889864 |
| IL-4 (3) | 3.02E-05 | 0.860565 |
| VEGF (4) | 3.20E-05 | 0.848107 |
| IL-3 (2) | 3.35E-05 | 0.871146 |
| IL-2 (2) | 3.89E-05 | 0.843949 |
| IL-1ra (3) | 3.93E-05 | 0.88975 |
| RANTES (3) | 4.51E-05 | 0.933006 |
| CIMS (31) | 4.80E-05 | 0.886723 |
| CIMS (27) | 490E-05 | 0.915073 |
| TGF-β1 (3) | 4.91E-05 | 0.889071 |

**TABLE A**

| *Uniprot entry ID* | *Recommended protein name* | *Short name* | Diagnosis | |
|---|---|---|---|---|
| | | | *PC* | *PC (body&tail) vs PC (head)* |
| **(i) Core Biomarkers** | | | | |
| Q6IBS9 | HADH2 protein | HADH2 | X | |
| P36941 | Tumor necrosis factor receptor superfamily member 3 | TNFRSF3 | X | |

| **(ii) Preferred Biomarkers (PC & BTvH)** | | | | |
|---|---|---|---|---|
| P35716 | Transcription factor SOX-11 | Sox11A | X | X |
| O75578 | Integrin alpha-10 | Integrin α-10 | X | X |
| NA | EDFR | | X | X |
| NA | EPFR | | X | X |
| NA | LSADHR | | X | X |
| NA | SEAHLR | | X | X |
| NA | AQQHQWDGLLSYQDSLS | | X | X |
| NA | WTRNSNMNYWLIIRL | | X | X |
| NA | WDSR | | X | X |

| **(iii) Preferred Biomarkers (PC)** | | | | |
|---|---|---|---|---|
| NA | DFAEDK | | X | |
| Q6PJJ3 | FASN protein | FASN | X | |
| Q5U4P5 | GAK protein | GAK | X | |
| NA | LNVWGK | | X | |
| NA | LTEFAK | | X | |
| NA | LYEIAR | | X | |
| P42679 | Megakaryocyte-associated tyrosine-protein kinase | MATK | X | |
| Q9H4L5 | Oxysterol-binding protein-related protein 3 | ORP-3 | X | |
| NA | QEASFK | | X | |
| NA | SSAYSR | | X | |
| NA | QEASFK | | X | |
| NA | TEEQLK | | X | |
| NA | TLYVGK | | X | |
| NA | FLLMQYGGMDEHAR | | X | |
| NA | GIVKYLYEDEG | | X | |
| NA | GIVKYLYEDEG | | X | |
| P43405 | Tyrosine-protein kinase SYK | KSYK | X | |

| **(iv) Optional Biomarkers (PC & BTvH)** | | | | |
|---|---|---|---|---|
| Q4VCS5 | Angiomotin | Angiomotin | X | X |
| P13500 | C-C motif chemokine 2 | MCP-1 | X | X |
| P13501 | C-C motif chemokine 5 | RANTES | X | X |
| P29965 | CD40 ligand | CD40L | X | X |
| P01275 | Glucagon-like peptide-1 | GLP-1 | X | X |
| NA | Immunoglobilin M | IgM | X | X |
| P01583 | Interleukin-1 alpha | IL-1a | X | X |
| P18510 | Interleukin-1 receptor antagonist protein | IL-1ra | X | X |
| P20809 | Interleukin-11 | IL-11 | X | X |
| P29459/60 | Interleukin-12 | IL-12 | X | X |
| Q14005 | Interleukin-16 | IL-16 | X | X |
| Q14116 | Interleukin-18 | IL-18 | X | X |
| P60568 | Interleukin-2 | IL-2 | X | X |
| P08700 | Interleukin-3 | IL-3 | X | X |
| P05112 | Interleukin-4 | IL-4 | X | X |
| P05231 | Interleukin-6 | IL-6 | X | X |
| P13232 | Interleukin-7 | IL-7 | X | X |
| P15248 | Interleukin-9 | IL-9 | X | X |
| NA | Lewis x | Lewis x | X | X |
| P01374 | Lymphotoxin-alpha | TNF-b | X | X |
| P01137 | Transforming growth factor beta-1 | TGF-b1 | X | X |
| P15692 | Vascular endothelial growth factor | VEGF | X | X |
| P58304 | Visual system homeobox 2 | CHX10 | X | X |
| P01903/P01911/ P79483/P13762/ Q30154/P20036/ P04440 | | HLA-DR/DP | X | X |

| **(v) Optional Biomarkers (PC)** | | | | |
|---|---|---|---|---|
| P02647 | Apolipoprotein A1 | Apo-A1 | X | |
| P06727 | Apolipoprotein A4 | Apo-A4 | X | |
| P04114 | Apolipoprotein B-100 ATP synthase subunit beta, | LDL | X | |
| P06576 | mitochondrial | ATP-5B | X | |
| P16278 | Beta-galactosidase | B-galactosidase | X | |
| P56202 | Cathepsin W | Procathepsin W | X | |
| Q99616 | C-C motif chemokine 13 | MCP-4 | X | |
| P80098 | C-C motif chemokine 7 | MCP-3 | X | |
| Q6P2H9 | CD40 protein | CD40 | X | |
| P02745/6/7 | Complement C1q | C1q | X | |
| P09871 | Complement C1s | C1s | X | |
| P01024 | Complement C3 | C3 | X | |
| P0COL4/5 | Complement C4 | C4 | X | |
| P01031 | Complement C5 | C5 | X | |
| P00751 | Complement factor B | Factor B | X | |
| P24941 | Cyclin-dependent kinase 2 | CDK-2 | X | |
| P01034 | Cystatin-C | Cystatin C | X | |
| P51671 | Eotaxin | Eotaxin | X | |
| P00533 | Epidermal growth factor receptor | EGFR | X | |
| P43220 | Glucagon-like peptide 1 receptor | GLP-1R | X | |
| P04141 | Granulocyte-macrophage colony-stimulating factor | GM-CSF | X | |
| Q9UKX5 | Integrin alpha-11 | Integrin a-11 | X | |
| P05362 | Intercellular adhesion molecule 1 | ICAM-1 | X | |
| P01579 | Interferon gamma | IFN-g | X | |
| P01584 | Interleukin-1 beta | IL-lb | X | |
| P22301 | Interleukin-10 | IL-10 | X | |
| P35225 | Interleukin-13 | IL-13 | X | |
| P05113 | Interleukin-5 | IL-5 | X | |
| P10145 | Interleukin-8 | IL-8 | X | |
| P08727 | Keratin, type I cytoskeletal 19 | Keratin19 | X | |
| P41159 | Leptin | Leptin | X | |
| P51884 | Lumican | Lumican | X | |
| P28482 | Mitogen-activated protein kinase 1 | MAPK1 | X | |
| P45983 | Mitogen-activated protein kinase 8 | MAPK8 | X | |
| P15941 | Mucin-1 | MUC-1 | X | |
| P54296 | Myomesin-2 | Myomesin-2 | X | |
| P10451 | Osteopontin Phosphatidylinositol 3-kinase | Osteopontin | X | |
| P27986 | regulatory subunit alpha | P85A | X | |
| P05155 | Plasma protease C1 inhibitor | C1 inh | X | |
| P27918 | Properdin | Properdin | X | |
| P07288 | Prostate-specific antigen | PSA | X | |
| P04626 | Receptor tyrosine-protein kinase erbB-2 | Her2/ErbB-2 | X | |
| Q9BZI7 | Regulator of nonsense transcripts 3B | UPF3B | X | |
| Q15349 | Ribosomal protein S6 kinase alpha-2 | RPS6KA2 | X | |
| NA | Sialyl Lewis x | Sialyl Lewis x | X | |
| Q9UGK3 | Signal-transducing adaptor protein 2 | STAP2 | X | |
| P63279 | SUMO-conjugating enzyme UBC9 | UBC9 | X | |
| Q6ZT07 | TBC1 domain family member 9 | TBC1D9 | X | |
| NA | Transmembrane peptide | (Tm peptide) | X | |
| P01375 | Tumor necrosis factor alpha | TNF-a | X | |
| Q92956 | Tumor necrosis factor receptor superfamily member 14 | TNFRSF14 | X | |
| Q06187 | Tyrosine-protein kinase BTK | BTK | X | |
| P52333 | Tyrosine-protein kinase JAK3 | JAK3 | X | |
| P18031 | Tyrosine-protein phosphatase non-receptor type 1 | PTP-1B | X | |
| Q9Y5K5 | Ubiquitin carboxyl-terminal hydrolase isozyme L5 | UCHL5 | X | |
| O00762 | Ubiquitin-conjugating enzyme E2 C | UBE2C | X | |

| **(vi) Preferred Biomarkers (BTvH)** | | | | |
|---|---|---|---|---|
| NA | FIQTDK | | | X |

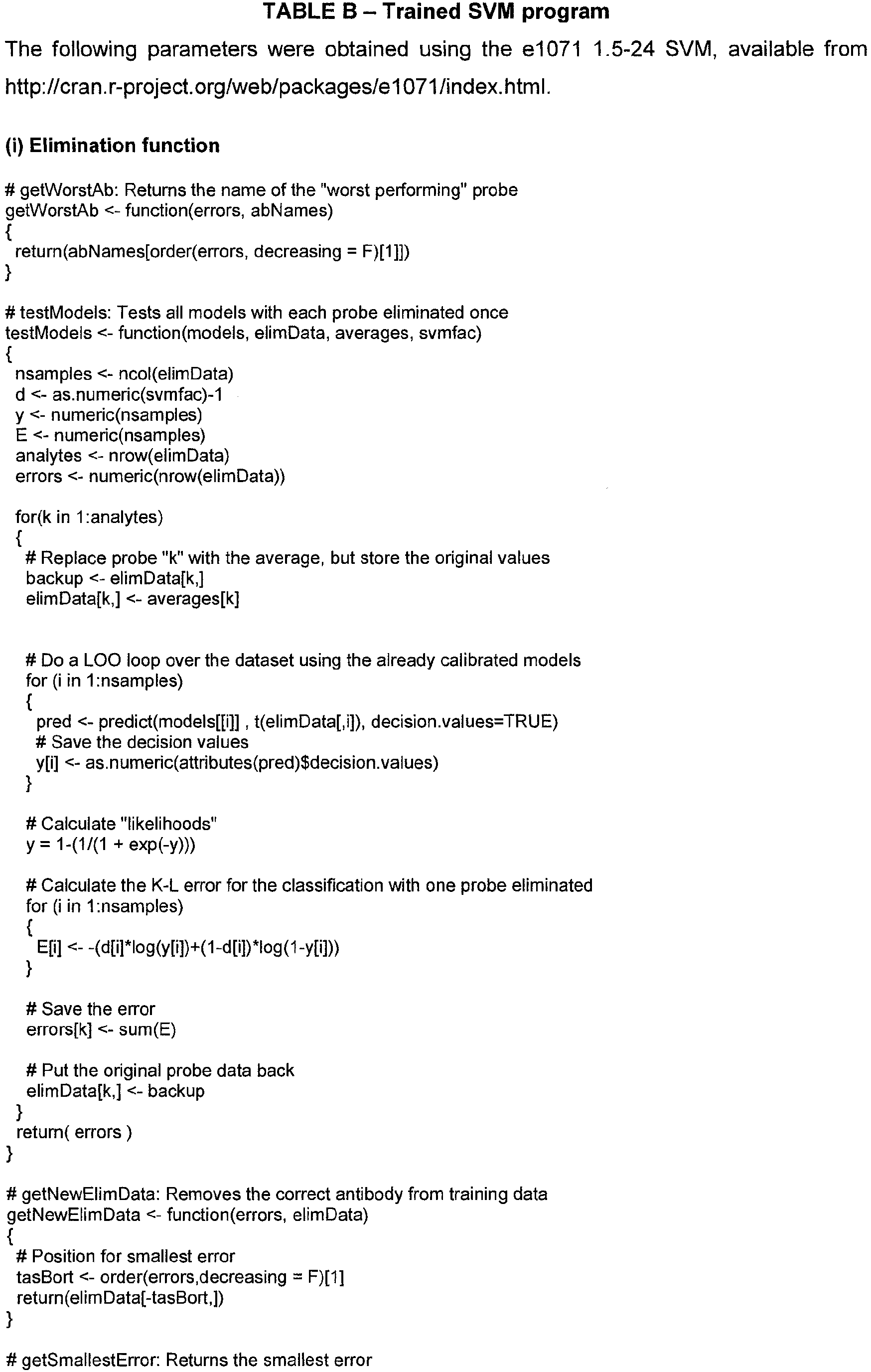

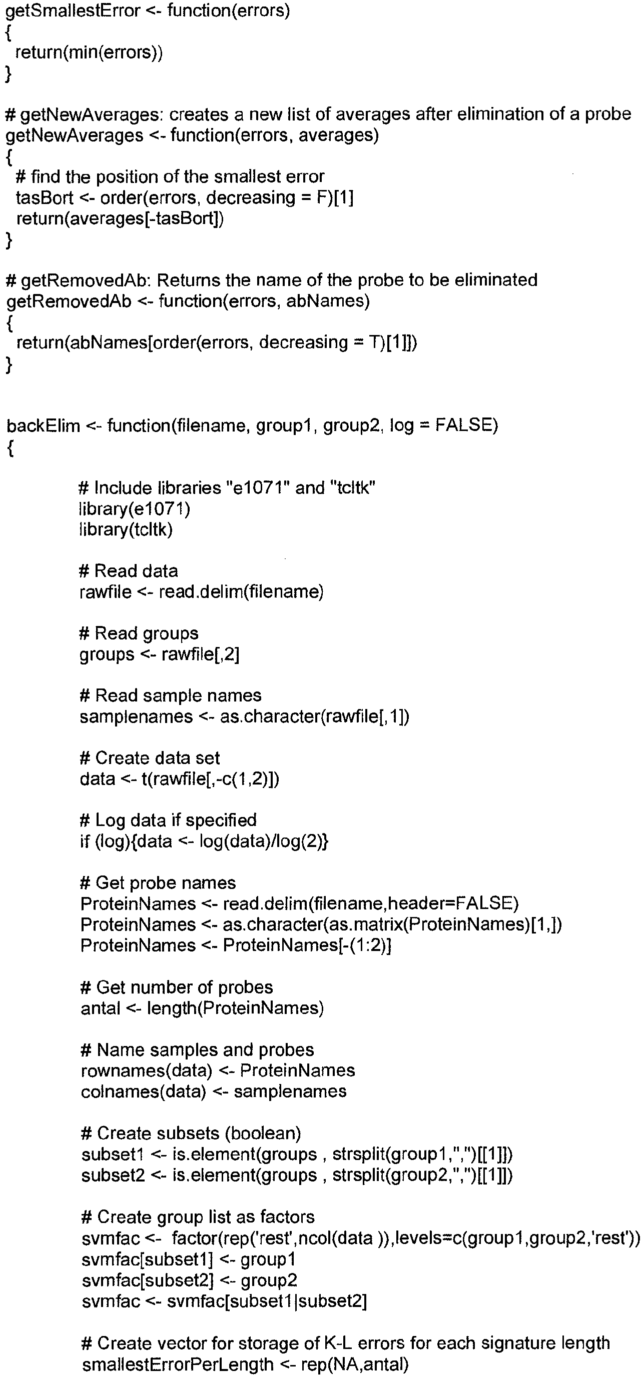

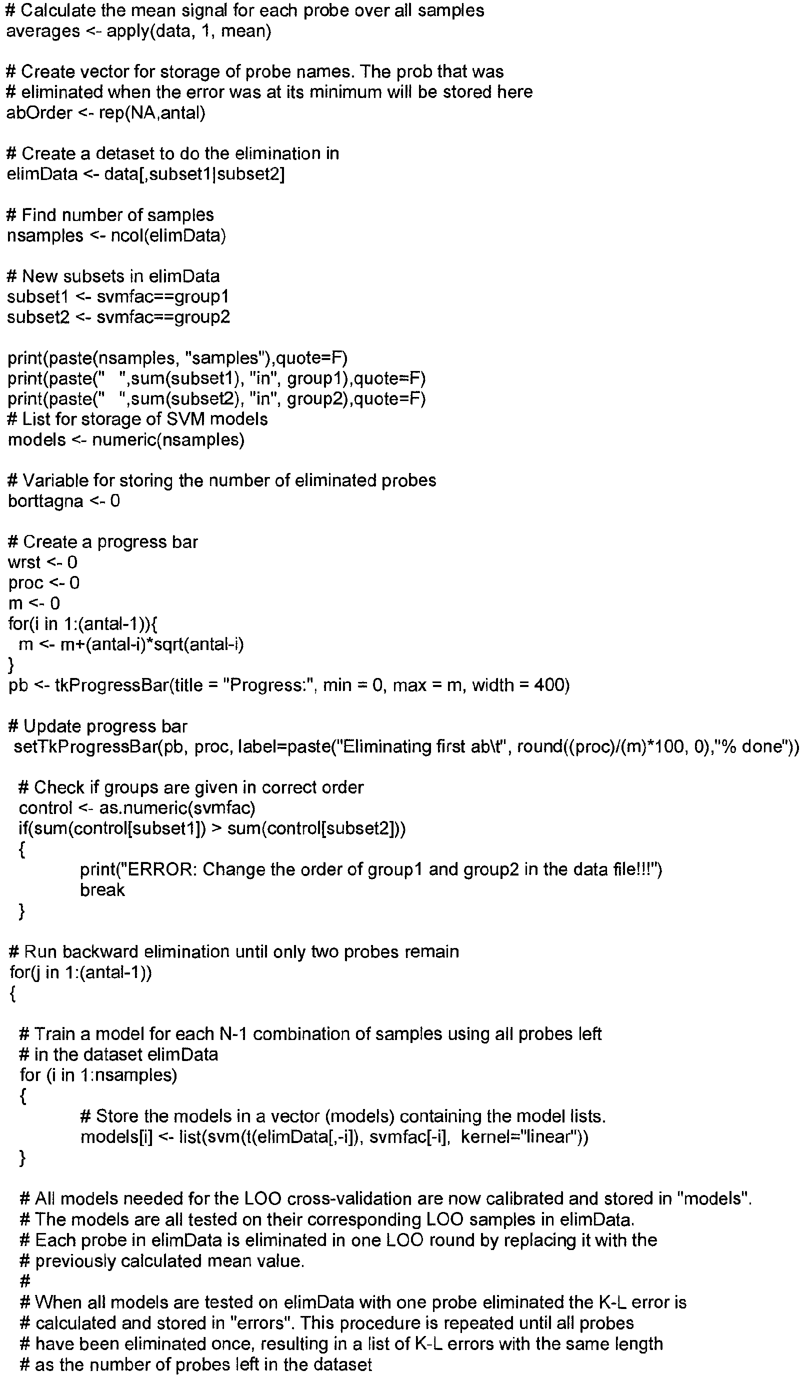

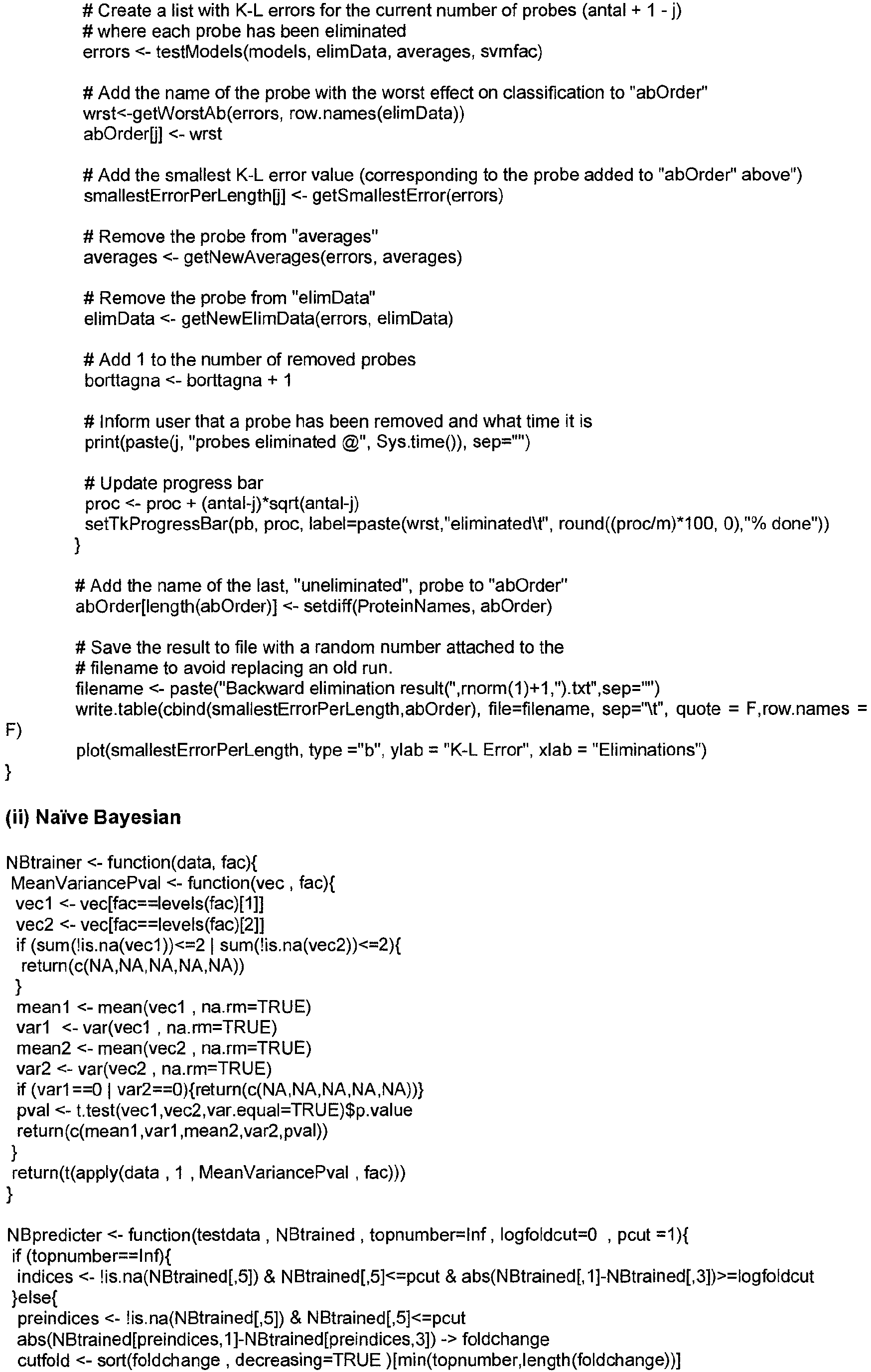

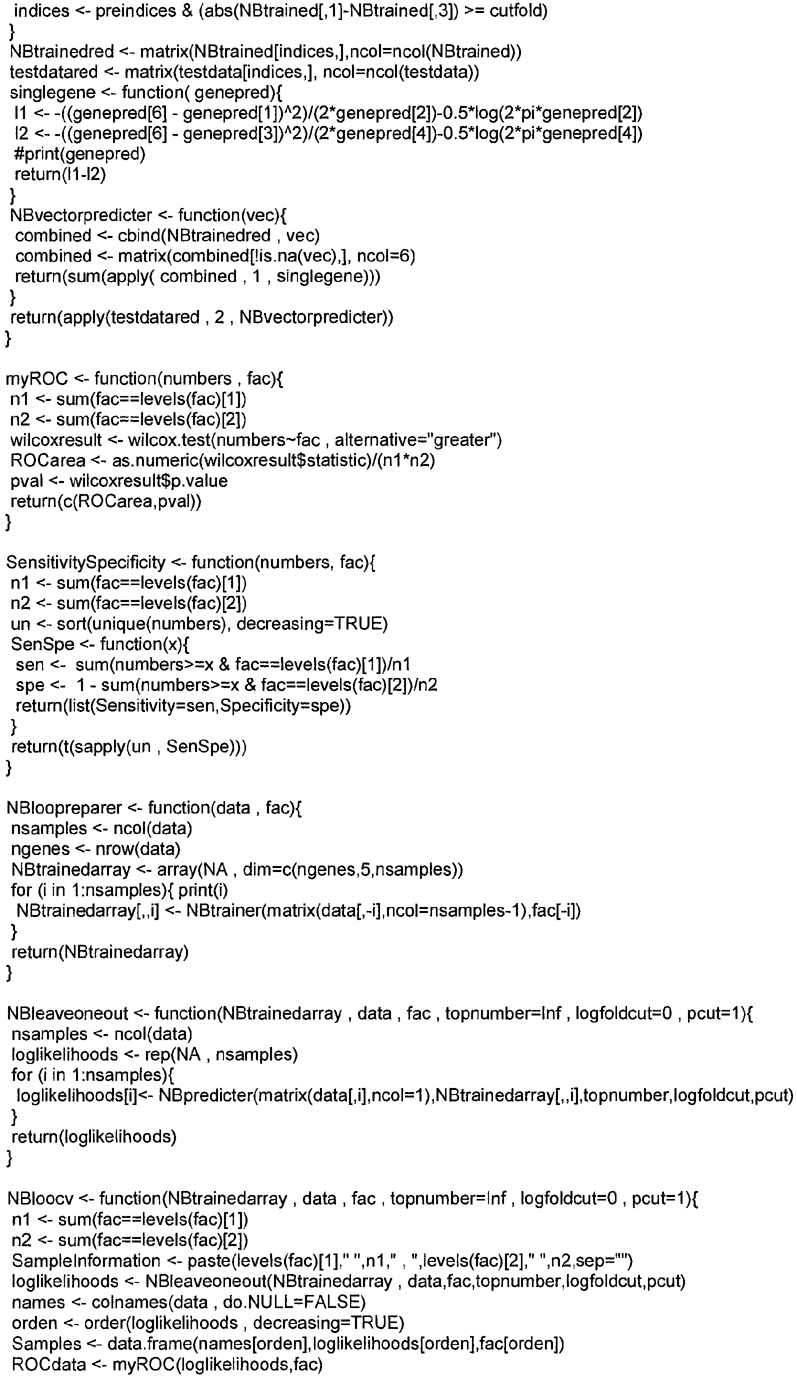

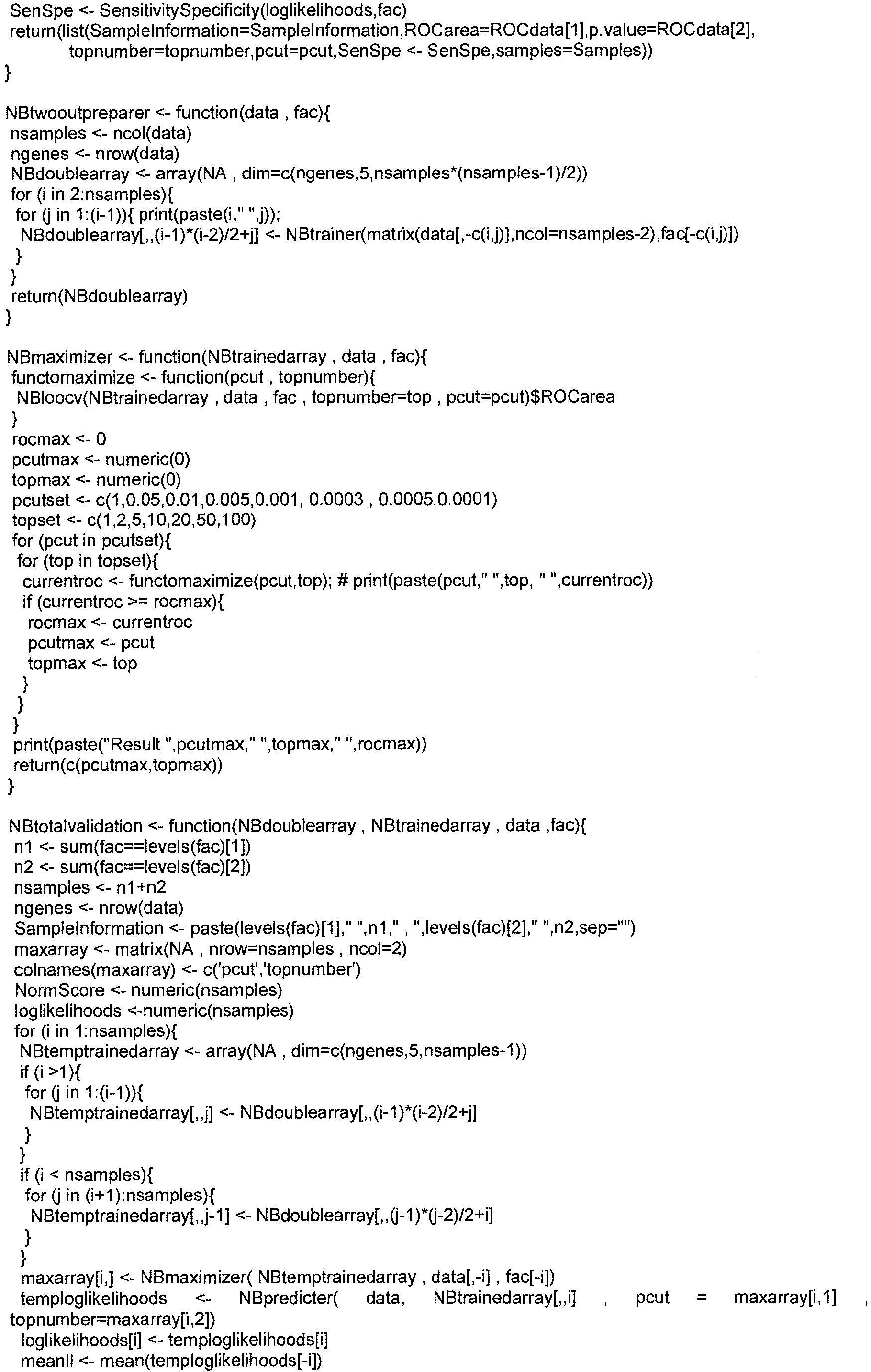

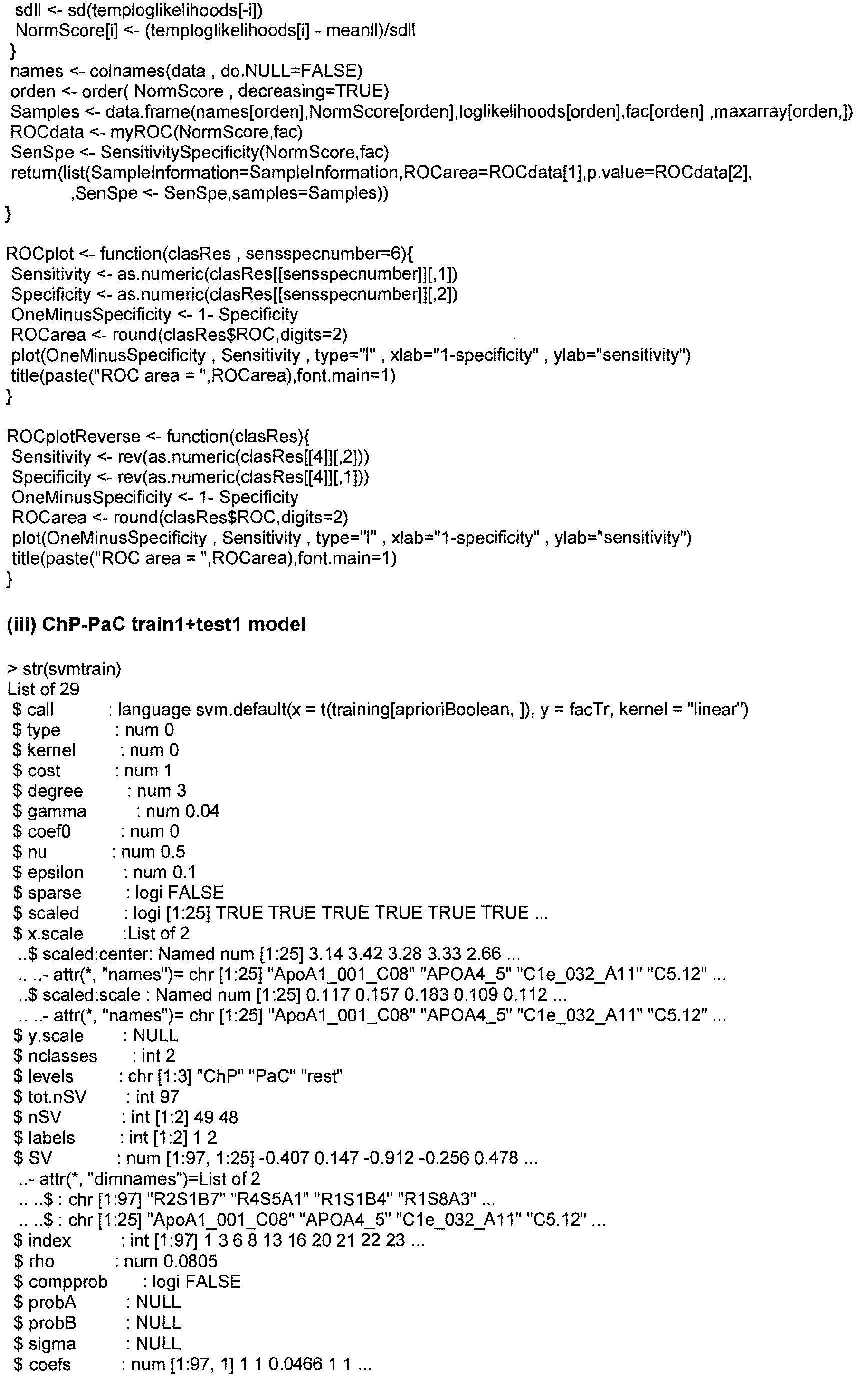

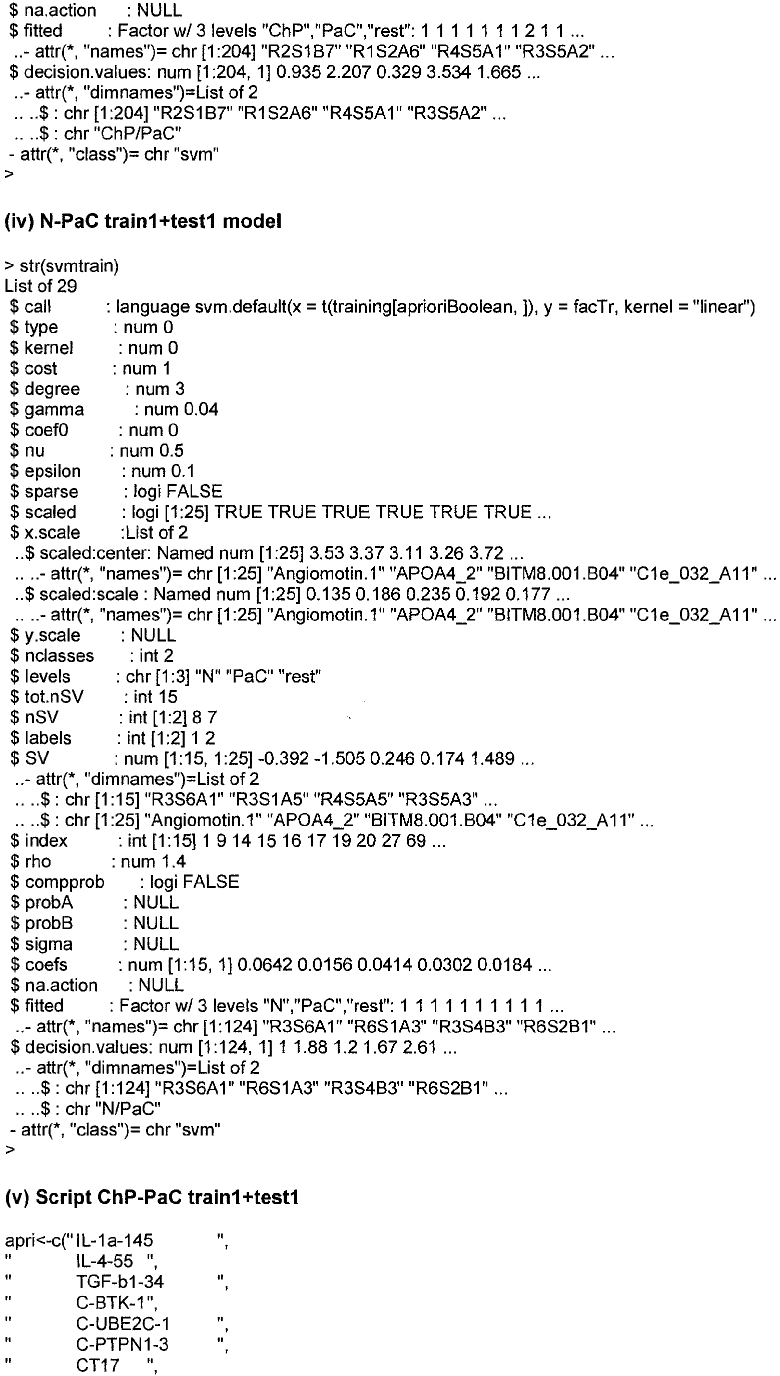

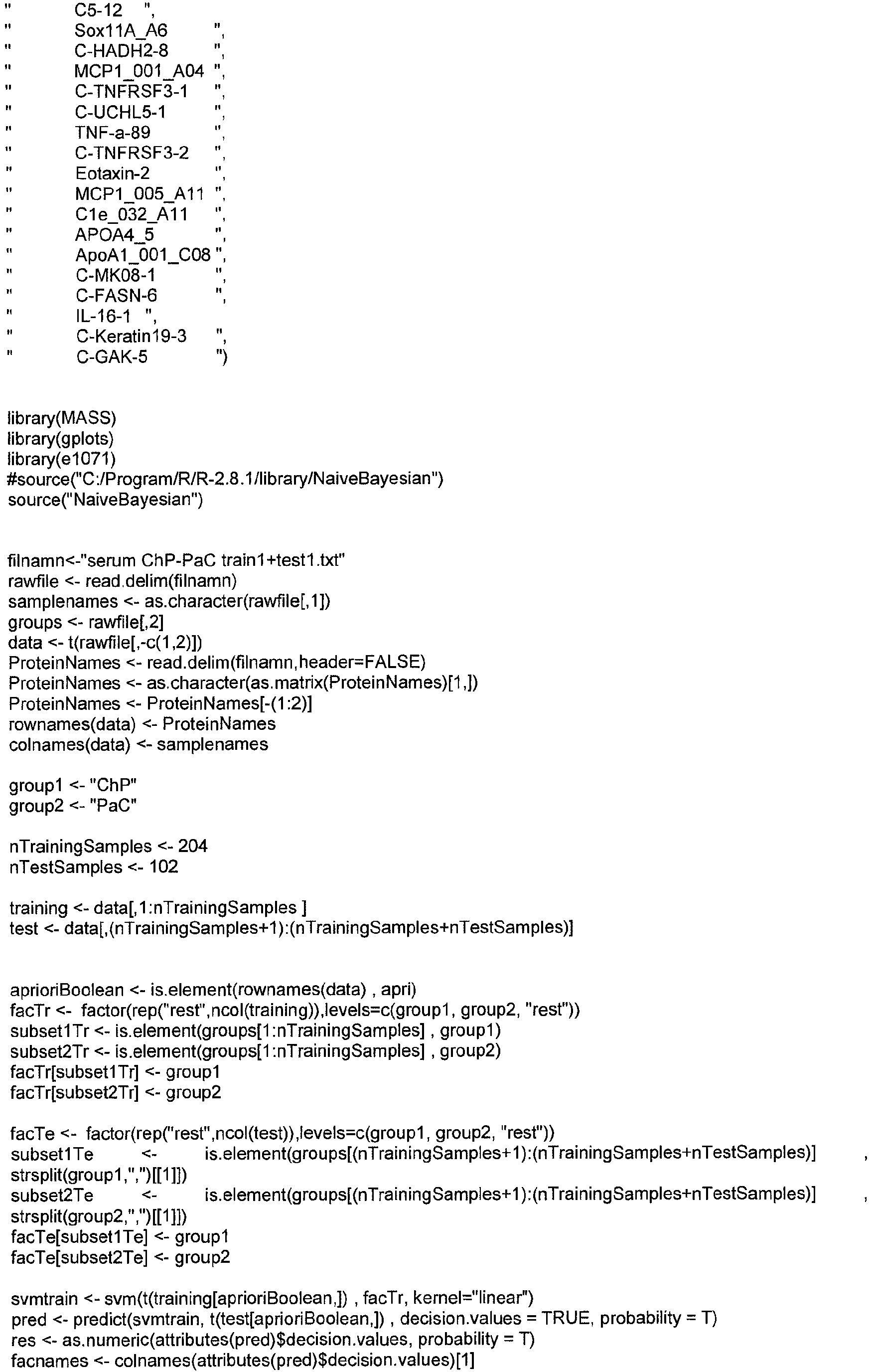

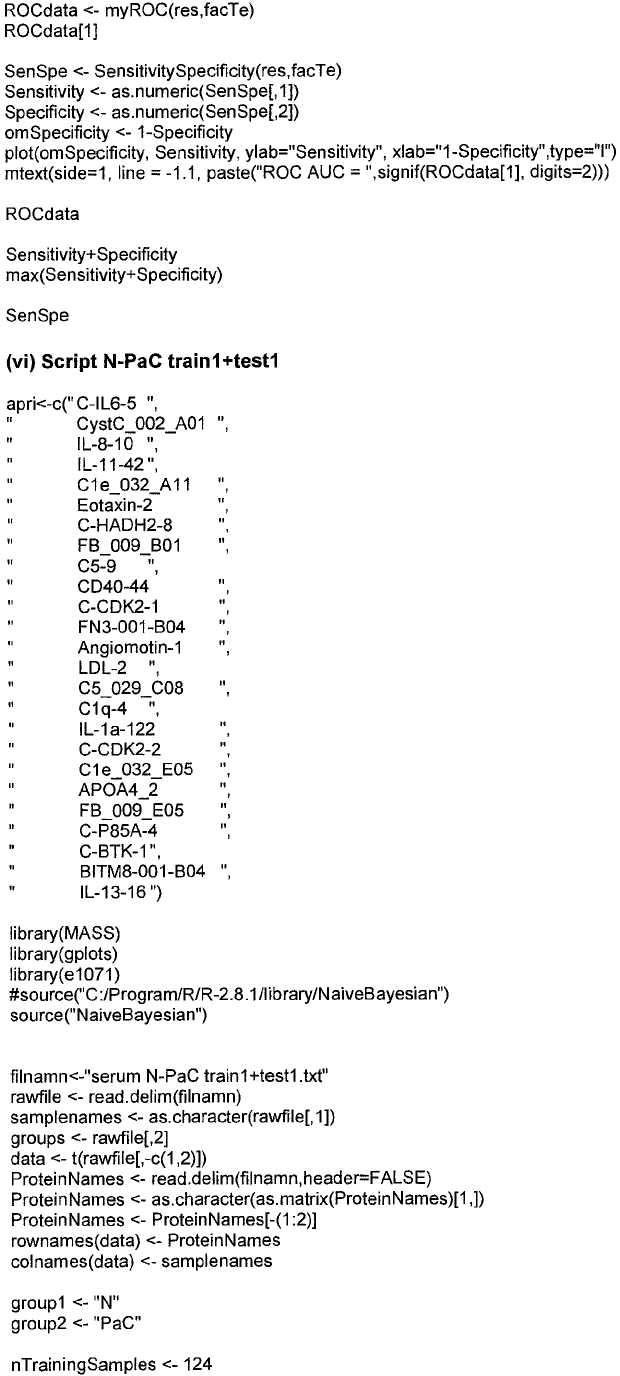

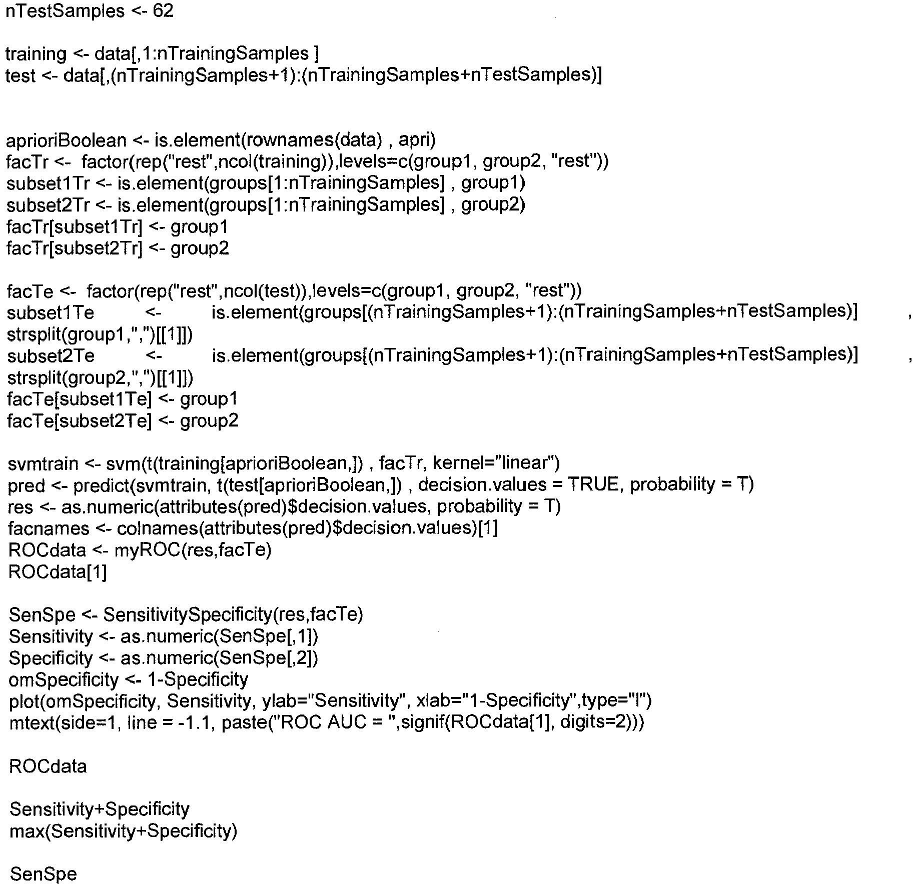

**Table C - Exemplary discriminating power of biomarkers and biomarker combinations (PC v NC)**

| **ROC-AUC** | **Biomarker signature** |
|---|---|
| 0.9 | HADH2 |
| 0.83 | TNFRSF3 |
| 0.93 | HADH2+TNFRSF3 |
| 0.96 | HADH2+TNFRSF3+GAK |
| 0.95 | HADH2+TNFRSF3+GAK+UPF3B |
| 0.95 | HADH2+TNFRSF3+GAK+UPF3B+Integrin a-10 |

## Claims

1. A method for determining the presence of pancreatic cancer in an individual comprising or consisting of the steps of:
a) providing a sample to be tested obtained from the individual;
b) determining a biomarker signature of the test sample by measuring the expression in the test sample of two or more biomarkers selected from the group defined in Table A;
wherein step (b) comprises measuring the expression in the test sample of HADH2, and
wherein the expression in the test sample of the two or more biomarkers selected from the group defined in Table A is indicative of the presence of pancreatic cancer.

2. The method according to Claim 1 further comprising or consisting of the steps of:
c) providing a control sample obtained from an individual not afflicted with pancreatic cancer;
d) determining a biomarker signature of the control sample by measuring the expression in the control sample of the two or more biomarkers measured in step (b);
wherein the presence of pancreatic cancer is identified in the event that the expression in the test sample of the two or more biomarkers measured in step (b) is different from the expression in the control sample of the two or more biomarkers measured in step (d).

3. The method according to Claim 2 further comprising or consisting of the steps of:
e) providing a control sample obtained from an individual afflicted with pancreatic cancer;
f) determining a biomarker signature of the control sample by measuring the expression in the control sample of the two or more biomarkers measured in step (b);
wherein the presence of pancreatic cancer is identified in the event that the expression in the test sample of the two or more biomarkers measured in step (b) corresponds to the expression in the control sample of the two or more biomarkers measured in step (f).

4. The method according to any one of the preceding claims, wherein step (b) further comprises measuring the expression of
(a) TNFRSF3;
(b) one or more of the biomarkers listed in Table (A)(ii);
(c) one or more biomarkers from the biomarkers listed in Table A(iii);
(d) one or more biomarkers from the biomarkers listed in Table A(iv);
(e) one or more biomarkers from the biomarkers listed in Table A(v) ; and/or
(f) one or more biomarkers from the biomarkers listed in Table A(vi).

5. The method according to any one of the preceding claims wherein step (b) comprises or consists of measuring the expression in the test sample of all of the biomarkers defined in Table A.

6. The method according to any one of the preceding claims wherein the pancreatic cancer is selected from the group consisting of adenocarcinoma, adenosquamous carcinoma, signet ring cell carcinoma, hepatoid carcinoma, colloid carcinoma, undifferentiated carcinoma, and undifferentiated carcinomas with osteoclast-like giant cells.

7. The method according to any one of the preceding claims wherein step (b), (d) and/or step (f) is performed using first binding agents capable of binding to the two or more biomarkers, wherein the first binding agents each comprise or consist of an antibody or an antigen-binding fragment thereof.

8. The method according to any one of the preceding claims wherein the two or more biomarkers in the test sample and/or the control sample are labelled with a detectable moiety.

9. The method according to any one of Claims 7 or 8 wherein step (b), (d) and/or step (f) is performed using an assay comprising second binding agents capable of binding to the two or more biomarkers, the second binding agents each comprising a detectable moiety, wherein the second binding agents each comprise or consist of an antibody or an antigen-binding fragment thereof.

10. The method according to any one of the preceding claims wherein the method comprises:
(i) labelling biomarkers present in the sample with biotin;
(ii) contacting the biotin-labelled proteins with an array comprising a plurality of scFv immobilised at discrete locations on its surface, the scFv having specificity for one or more of the proteins in Table A;
(iii) contacting the immobilised scFv with a streptavidin conjugate comprising a fluorescent dye; and
(iv) detecting the presence of the dye at discrete locations on the array surface
wherein the expression of the dye on the array surface is indicative of the expression of a biomarker from Table A in the sample.

11. The method according to any one of Claims 1 to 6 wherein, step (b), (d) and/or (f) comprises measuring the expression of a nucleic acid molecule encoding the two or more biomarkers.

12. The method according to Claim 11, wherein measuring the expression of the two or more biomarker(s) in step (b), (d) and/or (f) is performed using two or more binding moieties, each individually capable of binding selectively to a nucleic acid molecule encoding one of the biomarkers identified in Table A, wherein the one or more binding moieties each comprise or consist of a nucleic acid molecule.

13. The method according to any one of the preceding claims wherein, the sample provided in step (a), (c) and/or (e) is selected from the group consisting of unfractionated blood, plasma, serum, tissue fluid, pancreatic tissue, pancreatic juice, bile and urine.

14. Use of an array comprising two or more binding agents as defined in Claim 7 or 9 for determining the presence of pancreatic cancer in an individual, wherein the array comprises a binding agent capable of binding to HADH2, wherein the binding agents each comprise or consist of an antibody or an antigen-binding fragment thereof.

15. Use of two or more biomarkers selected from the group defined in Table A as diagnostic markers for determining the presence of pancreatic cancer in an individual *in vitro,* wherein the two or more biomarkers include HADH2.

## Patentansprüche

1. Verfahren zum Bestimmen des Vorhandenseins von Bauchspeicheldrüsenkrebs bei einem Individuum, umfassend die oder bestehend aus den folgenden Schritten:
a) Bereitstellen einer zu testenden Probe, die von dem Individuum erhalten wurde;
b) Bestimmen einer Biomarker-Signatur der Testprobe, indem die Expression von zwei oder mehr Biomarkern, ausgewählt aus der in Tabelle A definierten Gruppe, in der Testprobe gemessen wird;
wobei Schritt (b) das Messen der Expression von HADH2 in der Testprobe umfasst, und
wobei die Expression der zwei oder mehr Biomarker, ausgewählt aus der in Tabelle A definierten Gruppe, in der Testprobe das Vorhandensein von Bauchspeicheldrüsenkrebs anzeigt.

2. Verfahren nach Anspruch 1, des Weiteren umfassend die oder bestehend aus den folgenden Schritten:
c) Bereitstellen einer Kontrollprobe, die von einem Individuum erhalten wurde, das nicht an Bauchspeicheldrüsenkrebs leidet;
d) Bestimmen einer Biomarker-Signatur der Kontrollprobe, indem die Expression der zwei oder mehr Biomarker, die in Schritt (b) gemessen wurden, in der Kontrollprobe gemessen wird; wobei das Vorhandensein von Bauchspeicheldrüsenkrebs identifiziert ist, wenn sich die Expression der zwei oder mehr in Schritt (b) gemessenen Biomarker in der Testprobe von der Expression der zwei oder mehr in Schritt (d) gemessenen Biomarker in der Kontrollprobe unterscheidet.

3. Verfahren nach Anspruch 2, des Weiteren umfassend die oder bestehend aus den folgenden Schritten:
e) Bereitstellen einer Kontrollprobe, die von einem Individuum erhalten wurde, das an Bauchspeicheldrüsenkrebs leidet;
f) Bestimmen einer Biomarker-Signatur der Kontrollprobe, indem die Expression der zwei oder mehr Biomarker, die in Schritt (b) gemessen wurden, in der Kontrollprobe gemessen wird; wobei das Vorhandensein von Bauchspeicheldrüsenkrebs identifiziert ist, wenn die Expression der zwei oder mehr in Schritt (b) gemessenen Biomarker in der Testprobe der Expression der zwei oder mehr in Schritt (f) gemessenen Biomarker in der Kontrollprobe entspricht.

4. Verfahren nach einem der vorhergehenden Ansprüche, wobei Schritt (b) des Weiteren das Messen der Expression von
(a) TNFRSF3;
(b) einem oder mehreren der in Tabelle (A) (ii) aufgeführten Biomarker;
(c) einem oder mehreren Biomarkern der in Tabelle (A) (iii) aufgeführten Biomarker;
(d) einem oder mehreren Biomarkern der in Tabelle (A) (iv) aufgeführten Biomarker;
(e) einem oder mehreren Biomarkern der in Tabelle (A) (v) aufgeführten Biomarker; und/oder
(f) einem oder mehreren Biomarkern der in Tabelle (A) (vi) aufgeführten Biomarker umfasst.

5. Verfahren nach einem der vorhergehenden Ansprüche, wobei Schritt (b) das Messen der Expression aller in Tabelle A definierten Biomarker in der Testprobe umfasst oder daraus besteht.

6. Verfahren nach einem der vorhergehenden Ansprüche, wobei der Bauchspeicheldrüsenkrebs aus der Gruppe bestehend aus Adenokarzinom, adenosquamösem Karzinom, Siegelringkarzinom, hepatoidem Karzinom, kolloidalem Karzinom, undifferenziertem Karzinom und undifferenzierten Karzinomen mit osteoklastenähnlichen Riesenzellen ausgewählt ist.

7. Verfahren nach einem der vorhergehenden Ansprüche, wobei Schritt (b), (d) und/oder Schritt (f) unter Verwendung erster Bindungsmittel durchgeführt wird, die an die zwei oder mehr Biomarker binden können, wobei die ersten Bindungsmittel jeweils einen Antikörper oder ein antigenbindendes Fragment davon umfassen oder daraus bestehen.

8. Verfahren nach einem der vorhergehenden Ansprüche, wobei die zwei oder mehr Biomarker in der Testprobe und/oder der Kontrollprobe mit einer nachweisbaren Einheit markiert sind.

9. Verfahren nach einem der Ansprüche 7 oder 8, wobei Schritt (b), (d) und/oder Schritt (f) unter Verwendung eines Assays durchgeführt wird, das zweite Bindungsmittel umfasst, die an die zwei oder mehr Biomarker binden können, wobei die zweiten Bindungsmittel jeweils eine nachweisbare Einheit umfassen, wobei die zweiten Bindungsmittel jeweils einen Antikörper oder ein antigenbindendes Fragment davon umfassen oder daraus bestehen.

10. Verfahren nach einem der vorhergehenden Ansprüche, wobei das Verfahren Folgendes umfasst:
(i) Markieren der in der Probe vorhandenen Biomarker mit Biotin;
(ii) Inkontaktbringen der mit Biotin markierten Proteine mit einer Anordnung, die eine Vielzahl von scFv umfasst, die an diskreten Stellen auf deren Oberfläche immobilisiert sind, wobei die scFv eine Spezifizität für eines oder mehrere der Proteine in Tabelle A aufweisen;
(iii) Inkontaktbringen der immobilisierten scFv mit einem Streptavidin-Konjugat, das einen Fluoreszenzfarbstoff umfasst; und
(iv) Nachweisen des Vorhandenseins des Farbstoffs an diskreten Stellen auf der Anordnungsoberfläche,
wobei die Expression des Farbstoffs auf der Anordnungsoberfläche die Expression eines Biomarkers aus Tabelle A in der Probe anzeigt.

11. Verfahren nach einem der Ansprüche 1 bis 6, wobei Schritt (b), (d) und/oder (f) das Messen der Expression eines Nukleinsäuremoleküls umfasst, das für die zwei oder mehr Biomarker kodiert.

12. Verfahren nach Anspruch 11, wobei das Messen der Expression der zwei oder mehr Biomarker in Schritt (b), (d) und/oder (f) unter Verwendung von zwei oder mehr Bindungseinheiten durchgeführt wird, die jeweils einzeln in der Lage sind, selektiv an ein Nukleinsäuremolekül zu binden, das für einen der in Tabelle A identifizierten Biomarker kodiert, wobei die eine oder mehreren Bindungseinheiten jeweils ein Nukleinsäuremolekül umfassen oder daraus bestehen.

13. Verfahren nach einem der vorhergehenden Ansprüche, wobei die in Schritt (a), (c) und/oder (e) bereitgestellte Probe aus der Gruppe bestehend aus nicht fraktioniertem Blut, Plasma, Serum, Gewebefluid, Bauchspeicheldrüsengewebe, Pankreassaft, Galle und Urin ausgewählt ist.

14. Verwendung einer Anordnung, umfassend zwei oder mehr Bindungsmittel wie in Anspruch 7 oder 9 definiert, zum Bestimmen des Vorhandenseins von Bauchspeicheldrüsenkrebs bei einem Individuum, wobei die Anordnung ein Bindungsmittel umfasst, das an HADH2 binden kann, wobei die Bindungsmittel jeweils einen Antikörper oder ein antigenbindendes Fragment davon umfassen oder daraus bestehen.

15. Verwendung von zwei oder mehr Biomarkern, ausgewählt aus der Gruppe definiert in Tabelle A, als diagnostische Marker zum Bestimmen des Vorhandenseins von Bauchspeicheldrüsenkrebs bei einem Individuum *in vitro,* wobei die zwei oder mehr Biomarker HADH2 enthalten.

## Revendications

1. Procédé pour déterminer la présence d'un cancer du pancréas chez un individu comprenant ou contenant les étapes de :
a) fourniture d'un échantillon à tester obtenu auprès de l'individu ;
b) détermination d'une signature de biomarqueur de l'échantillon de test en mesurant l'expression dans l'échantillon de test de deux biomarqueurs ou plus sélectionnés dans le groupe défini dans le Tableau A ;
l'étape (b) comprenant la mesure de l'expression dans l'échantillon de test de HADH2, et l'expression dans l'échantillon de test des deux ou plusieurs biomarqueurs sélectionnés dans le groupe défini dans le Tableau A indiquant la présence d'un cancer du pancréas.

2. Procédé selon la revendication 1 comprenant ou contenant en outre les étapes de :
c) fourniture d'un échantillon de commande obtenu auprès d'un individu non atteint d'un cancer du pancréas ;
d) détermination d'une signature de biomarqueur de l'échantillon de commande en mesurant l'expression dans l'échantillon de commande des deux ou plus biomarqueurs mesurée à l'étape (b)
la présence d'un cancer du pancréas étant identifiée dans le cas où l'expression dans l'échantillon de test des deux ou plus biomarqueurs mesurée à l'étape (b) est différente de l'expression dans l'échantillon de commande des deux ou plus biomarqueurs mesurée à l'étape (d).

3. Procédé selon la revendication 2 comprenant ou contenant en outre les étapes de :
(e) fourniture d'un échantillon de commande obtenu auprès d'un individu atteint d'un cancer du pancréas ;
(f) détermination d'une signature de biomarqueur de l'échantillon de commande en mesurant l'expression dans l'échantillon de commande des deux ou plus biomarqueurs mesurée à l'étape (b) ;
la présence d'un cancer du pancréas étant identifiée dans le cas où l'expression dans l'échantillon de test des deux ou plus biomarqueurs mesurée à l'étape (b) correspond à l'expression dans l'échantillon de commande des deux ou plus biomarqueurs mesurée à l'étape (f).

4. Procédé selon l'une quelconque des revendications précédentes, l'étape (b) comprenant en outre la mesure de l'expression de :
(a) TNFRSF3 ;
(b) un ou plusieurs des biomarqueurs listés dans le Tableau (A)(ii) ;
(c) un ou plusieurs biomarqueurs des biomarqueurs listés dans le Tableau A(iii) ;
(d) un ou plusieurs biomarqueurs des biomarqueurs listés dans le Tableau A(iv) ;
(e) un ou plusieurs biomarqueurs des biomarqueurs listés dans le Tableau A(v) ; et/ou
(f) un ou plusieurs biomarqueurs des biomarqueurs listés dans le Tableau A(vi).

5. Procédé selon l'une quelconque des revendications précédentes, l'étape (b) comprenant ou contenant la mesure de l'expression dans l'échantillon de test de l'ensemble des biomarqueurs définis dans le Tableau A.

6. Procédé selon l'une quelconque des revendications précédentes, le cancer du pancréas étant sélectionné dans le groupe constitué de l'adénocarcinome, du carcinome adénosquameux, du carcinome à cellules en bague à chaton, du carcinome hépatoïde, du carcinome colloïde, du carcinome indifférencié et des carcinomes indifférenciés avec des cellules géantes de type ostéoclaste.

7. Procédé selon l'une quelconque des revendications précédentes, l'étape (b), (d) et/ou l'étape (f) étant réalisée(s) à l'aide de premiers agents de liaison pouvant se lier aux deux ou plus biomarqueurs, les premiers agents de liaison comprenant ou contenant chacun un anticorps ou un fragment de liaison d'antigène de celui-ci.

8. Procédé selon l'une quelconque des revendications précédentes, les deux ou plus biomarqueurs dans l'échantillon de test et/ou l'échantillon de commande étant marqués avec une fraction détectable.

9. Procédé selon l'une quelconque des revendications 7 ou 8, l'étape (b), (d) et/ou l'étape (f) étant réalisée(s) à l'aide d'un ensemble comprenant des seconds agents de liaison pouvant se lier aux deux ou plus biomarqueurs, les seconds agents de liaison comprenant chacun une fraction détectable, les seconds agents de liaison comprenant ou contenant chacun un anticorps ou un fragment de liaison d'antigène de celui-ci.

10. Procédé selon l'une quelconque des revendications précédentes, le procédé comprenant :
(i) le marquage des biomarqueurs présents dans l'échantillon avec de la biotine ;
(ii) la mise en contact des protéines marquées avec de la biotine avec un ensemble comprenant une pluralité de scFv immobilisés à des emplacements distincts sur sa surface, les scFv ayant une spécificité pour une ou plusieurs des protéines du Tableau A ;
(iii) la mise en contact des scFv immobilisés avec un conjugué de streptavidine comprenant un colorant fluorescent ; et
(iv) la détection de la présence du colorant à des emplacements distincts sur la surface de l'ensemble
l'expression du colorant sur la surface de l'ensemble indiquant l'expression d'un biomarqueur du Tableau A dans l'échantillon.

11. Procédé selon l'une quelconque des revendications 1 à 6, l'étape (b), (d) et/ou (f) comprenant la mesure de l'expression d'une molécule d'acide nucléique codant les deux ou plus biomarqueurs.

12. Procédé selon la revendication 11, la mesure de l'expression des deux ou plus biomarqueurs à l'étape (b), (d), et/ou (f) étant réalisée à l'aide de deux fractions de liaison ou plus, chacune pouvant individuellement se lier sélectivement à une molécule d'acide nucléique codant l'un des biomarqueurs identifiés dans le Tableau A, les une ou plusieurs fractions de liaison comprenant ou contenant chacune une molécule d'acide nucléique.

13. Procédé selon l'une quelconque des revendications précédentes, l'échantillon fourni à l'étape (a), (c) et/ou (e) étant sélectionné dans le groupe constitué du sang non fractionné, du plasma, du sérum, des tissus pancréatiques, du suc pancréatique, de la bile et de l'urine.

14. Utilisation d'un ensemble comprenant deux agents de liaison ou plus selon la revendication 7 ou 9 pour déterminer la présence d'un cancer du pancréas chez un individu, l'ensemble comprenant un agent de liaison pouvant se lier à HADH2, les agents de liaison comprenant ou contenant chacun un anticorps ou un fragment de liaison d'antigène de celui-ci.

15. Utilisation de deux biomarqueurs ou plus sélectionnés dans le groupe défini dans le Tableau A tels que les marqueurs de diagnostic pour déterminer la présence d'un cancer du pancréas chez un individu *in vitro,* les deux ou plus biomarqueurs comportant HADH2.
